(19) Europäisches Patentamt
European Patent Office
Office européen des brevets

(11) Veröffentlichungsnummer: **0 358 957**
**A1**

(12) # EUROPÄISCHE PATENTANMELDUNG

(21) Anmeldenummer: **89114943.7**

(22) Anmeldetag: **11.08.89**

(51) Int. Cl.5: **C07D 217/16 , C07D 471/04 , C07D 495/04 , C07D 471/14 , C07D 495/14 , A61K 31/47 , A61K 31/435 , //(C07D471/04, 221:00,209:00),(C07D471/04, 239:00,221:00),(C07D495/04, 333:00,221:00)**

(30) Priorität: **16.08.88 DE 3827727**

(43) Veröffentlichungstag der Anmeldung:
**21.03.90 Patentblatt 90/12**

(84) Benannte Vertragsstaaten:
**AT BE CH DE ES FR GB GR IT LI LU NL SE**

(71) Anmelder: **BOEHRINGER INGELHEIM KG**
**Postfach 200**
**D-6507 Ingelheim am Rhein(DE)**
(84) **BE CH DE ES FR GR IT LI LU NL SE AT**

Anmelder: **BOEHRINGER INGELHEIM INTERNATIONAL GmbH**
**Postfach 20**
**D-6507 Ingelheim am Rhein(DE)**
(84) **GB**

(72) Erfinder: **Lösel, Walter, Dr.**
**Im Herzenacker 26**
**D-6535 Gau-Algesheim(DE)**
Erfinder: **Roos, Otto, Dr.**
**Elsheimer Strasse 36**
**D-6501 Schwabenheim(DE)**
Erfinder: **Schnorrenberg, Gerd, Dr.**
**Ernst-Ludwig-Strasse 66a**
**D-6535 Gau-Algesheim(DE)**
Erfinder: **Arndts, Dietrich, Dr.**
**Mühlstrasse 7**
**D-6531 Appenheim(DE)**
Erfinder: **Speck, Georg, Dr.**
**Rheinstrasse 13**
**D-6507 Ingelheim am Rhein(DE)**
Erfinder: **Streller, Ilse, Dr.**
**Waldstrasse 16**
**D-6534 Stromberg(DE)**
Erfinder: **Traunecker, Werner, Dr.**
**Birkenweg 1**
**D-6538 Münster-Sarmsheim(DE)**

(54) **Anellierte Tetrahydropyridinessigsäurederivate, Verfahren zu deren Herstellung und Verwendung solcher Verbindungen zur Kardioprotektion.**

(57) Verbindungen der allgemeinen Formel I,

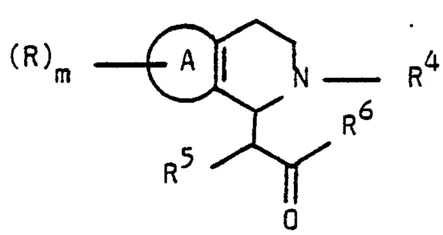

I

worin

EP 0 358 957 A1

A einen Benzo-, Thieno- oder Indolorest bedeutet,
$R^4$ Wasserstoff oder $(C_1$-$C_4)$Alkyl bedeutet,
$R^6$ Hydroxy, $(C_1$-$C_4)$Alkoxy oder eine $-NR^7R^8$-Gruppe bedeutet,
und R, m, $R^5$, $R^7$ und $R^8$ wie in den Ansprüchen definiert sind, zeigen cardioprotektive Wirkung.

Neue Ausgangsverbindungen für die Herstellung eines Teils der Verbindungen der Formel I, haben die allgemeine Formel II bzw. V

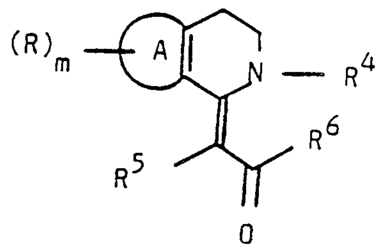

II

worin
A ein Indolrest ist,
R, m, $R^4$, $R^5$ und $R^6$ wie oben definiert sind.

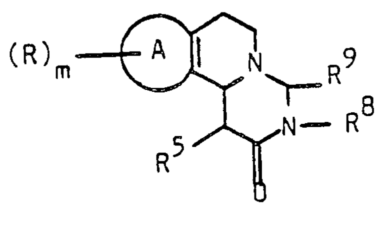

V

worin A, R, m, $R^5$, $R^8$ und $R^9$ wie in der Beschreibung definiert sind.

# Anellierte Tetrahydropyridinessigsäurederivate, Verfahren zu deren Herstellung und Verwendung solcher Verbindungen zur Kardioprotektion

Die Erfindung betrifft neue anellierte Tetrahydropyridinessigsäurederivate, Verfahren zu deren Herstellung, pharmazeutische Zubereitungen, die diese neuen oder strukturell nahestehende bekannte Verbindungen enthalten sowie die Verwendung der neuen Verbindungen oder strukturell nahestehender bekannter Verbindungen zur Kardioprotektion.

Ferner betrifft die Erfindung neue Zwischenprodukte für die Herstellung der obengenannten Verbindungen.

Die Erfindung betrifft die Verwendung einer Verbindung der allgemeinen Formel I

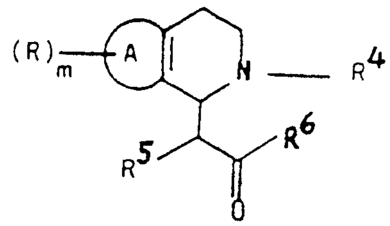

I

worin

A einen Benzo-, Thieno- oder Indolorest bedeutet;

R Wasserstoff, $(C_1-C_4)$Alkyl, Halogen (F, Cl, Br, J), Hydroxy, $(C_1-C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;

m 1, 2 oder 3 bedeutet, wenn A ein Benzo- oder Indolorest ist, und 1 oder 2 bedeutet, wenn A ein Thienorest ist;

$R^4$ Wasserstoff oder $(C_1-C_4)$Alkyl bedeutet;

$R^5$ Wasserstoff, $(C_1-C_{10})$Alkyl, Phenyl, Phenyl$(C_1-C_5)$alkyl, $(C_1-C_4)$Alkoxy oder -NHCOX (worin X $(C_1-C_5)$-Alkyl ist) bedeutet;

$R^6$ Hydroxy, $(C_1-C_4)$Alkoxy oder eine $-NR^7R^8$-Gruppe bedeutet,

worin $R^7$ und $R^8$ unabhängig voneinander

(a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeuten, wobei das Alkyl substituiert sein kann durch

Hydroxy,

$(C_1-C_4)$Alkoxy,

Di$(C_1-C_4)$Alkylamino,

Furyl,

Pyridyl,

Pyrrolidinyl,

Morpholino,

Indolyl,

Nitrilo,

Thienyl,

Phenyl oder Phenyl, das ein oder mehrfach durch Hydroxy, Methoxy oder Fluor substituiert ist; oder $R^7$ Wasserstoff bedeutet und $R^8$ Phenyl, Fluorophenyl, Pyridyl oder N-Benzylpiperidyl;

oder $R^7$ und $R^8$ gemeinsam mit dem Stickstoffatom, an das sie gebunden sind,

Pyrrolidinyl

Piperidinyl

Morpholinyl- oder

Piperazinyl bedeuten, wobei der Piperazinylring gegebenenfalls durch Methyl, unsubstituiertes Phenyl, Mono- oder Di$(C_1-C_4)$alkoxyphenyl, Pyrimidinyl oder Phenyl$(C_1-C_4)$alkyl N-substituiert sein kann;

oder deren pharmazeutisch annehmbaren Salzes mit einer anorganischen oder organischen Säure zur

Kardioprotektion.

Ferner betrifft die Erfindung eine pharmazeutische Zubereitung enthaltend eine Verbindung der Formel I, die wie oben definiert ist, oder deren pharmazeutisch annehmbare Salze mit anorganischen oder organischen Säuren,

ausgenommen

a) Verbindungen der Formel Ig,

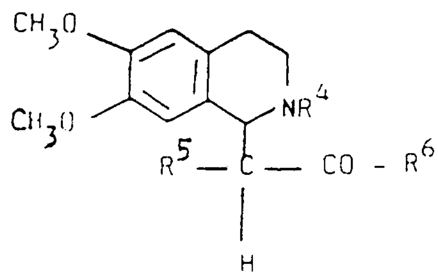

(Ig)

worin

$R^4$ H ist,

$R^5$ H ist und

$R^6$ $NH(CH_2)_3OH$, $NH(CH_2)_2OH$ oder $NH(CH_2)_3OCH_3$ ist;

b) Verbindungen der Formel I, worin

A ein Indolorest ist;

R Wasserstoff, $(C_1\text{-}C_4)$Alkyl, Halogen oder $(C_1\text{-}C_4)$Alkoxy bedeutet;

m 1 ist,

$R^4$ Wasserstoff oder $(C_1\text{-}C_4)$Alkyl bedeutet;

$R^5$ Wasserstoff bedeutet;

$R^6$ $(C_1\text{-}C_4)$Alkoxy oder eine $NHR^8$-Gruppe bedeutet, worin

$R^8$ Wasserstoff oder Alkyl mit 1 bis 4 Kohlenstoffatomen (das durch Phenyl substituiert sein kann) bedeutet; oder enthaltend eine Verbindung der allgemeinen Formel Ih,

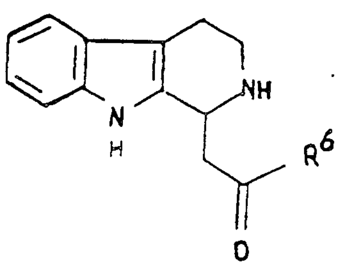

(Ih)

worin $R^6$ Ethoxy oder $-NHCH_2CH(CH_3)_2$ ist.

Die Erfindung betrifft ferner neue Verbindungen der Formel I

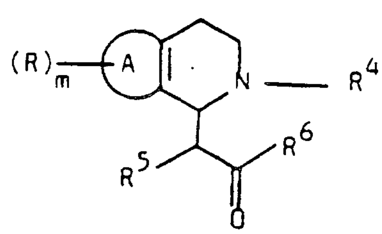

I

oder deren pharmazeutisch annehmbare Salze mit einer anorganischen oder organischen Säure, worin A, R, m, $R^4$, $R^5$ und $R^6$ wie oben definiert sind, ausgenommen

a) Verbindungen der Formel Ic,

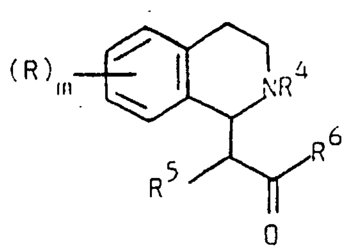

(Ic)

worin
R Wasserstoff, Hydroxy oder Methoxy ist,
m 1, 2 oder 3 ist,
$R^4$ und $R^5$ Wasserstoff sind und
$R^6$ Hydroxy oder Ethoxy ist;
sowie Verbindungen, worin
m 2 ist und
R Methoxy in den Positionen 6 und 7,
und
$R^4$ Wasserstoff, $R^5$ Ethyl und $R^6$ Methoxy ist,
oder
$R^4$ Methyl, $R^5$ Wasserstoff und $R^6$ Hydroxy oder Ethoxy ist
oder
$R^4$ Wasserstoff, $R^5$ Methyl und $R^6$ Ethoxy ist;
sowie Verbindungen der Formel Id,

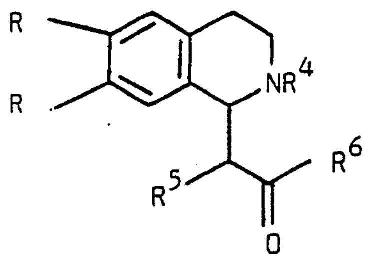

(Id)

worin R Wasserstoff oder Methoxy ist,
$R^4$ Methyl,
$R^5$ Wasserstoff oder Phenyl und
$R^6$ Ethoxy ist;

b) Verbindungen der Formel Ie,

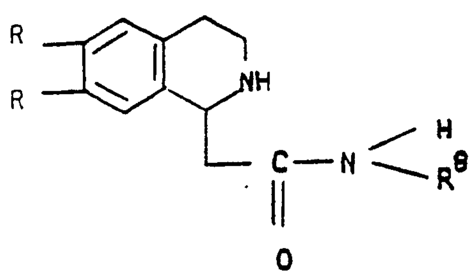

(Ie)

worin
R Wasserstoff, ($C_1$-$C_4$)Alkyl, Hydroxy oder ($C_1$-$C_4$)Alkoxy bedeutet, oder die zwei benachbarten Substituenten R zusammen -C-$CH_2$-O-sind,
$R^8$ Wasserstoff, Alkenyl, Alkyl, das durch Hydroxy, Methoxy, Dimethylamin oder Phenyl substituiert sein kann; bedeutet;

c) Verbindungen der Formel If,

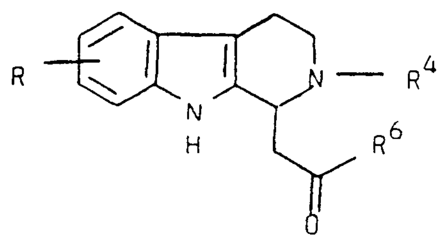

(If)

worin

R Wasserstoff, $(C_1-C_4)$Alkyl, Halogen oder $(C_1-C_4)$Alkoxy bedeutet,

$R^4$ Wasserstoff oder $(C_1-C_4)$Alkyl bedeutet;

$R^6$ $(C_1-C_4)$Alkoxy oder eine $-NHR^8$-Gruppe bedeutet,

worin

$R^8$ Wasserstoff oder $(C_1-C_4)$-Alkyl bedeutet,

wobei das Alkyl substituiert sein kann durch Phenyl.

Die allgemeine Formel I umfaßt auch die jeweiligen isomeren Formen der Verbindungen.

Einige der unter die allgemeine Definition der Formel I fallenden Verbindungen, in denen A ein Benzo- oder Indolorest ist, sind bekannt, manche davon sind als Tranquilizer beschrieben worden:

Eur. J. Med. Chem.- Chim. Ther, 14(1), 77-89

Heterocycles, 3(2), 179-82

Synthesis (5), 474-7

J. Med. Pharm. Chem. 3, 505-17 (1961)

EP 2 18 57

US 3 021 331

US 3 081 306

Es ist neu und überraschend, daß die Verbindungen der Formel I kardioprotektive Wirkung zeigen.

Die Herstellung der Verbindungen der allgemeinen Formel I kann nach an sich bekannten Verfahren erfolgen.

Eine Verbindung der Formel I, in der A, R, m, $R^4$, $R^5$ und $R^6$ wie oben definiert ist, kann hergestellt werden, indem die entsprechende Verbindung der allgemeinen Formel II

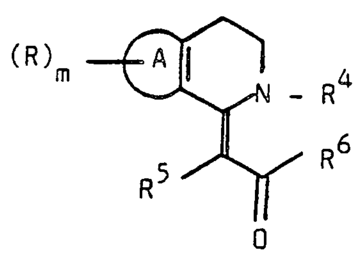

(II)

reduziert wird. In der Verbindung der Formel II sind A, R, m, $R^4$, $R^5$ und $R^6$ wie in der gewünschten Verbindung der Formel I definiert.

Vorzugsweise wird dieses Verfahren auf Verbindungen der Formel II angewendet, in denen $R^4$ Wasserstoff ist.

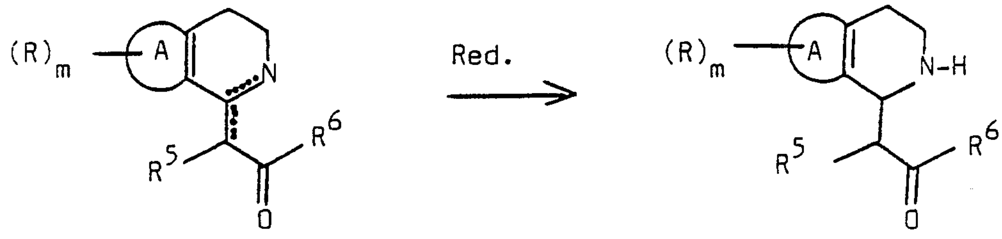


IIa Ia

Für die Herstellung einer Verbindung der Formel I, in der $R^4$ $(C_1$-$C_4)$Alkyl ist, kann eine der folgenden Varianten Anwendung finden:

a) eine Verbindung der Formel Ia wird N-alkyliert;

b) eine Verbindung der Formel IIa wird in ihr quaternäres Ammoniumsalz überführt: (z.B. durch Umsetzen mit $(C_1$-$C_4)$Alkyljodid) und anschließend zur Verbindung der Formel I (in der $R_4$ $(C_1$-$C_4)$Alkyl ist) reduziert.

Für die Reduktion der Verbindungen der Formel II bzw. IIa eignen sich komplexe Metallhydride (wie beispielsweise Natriumboranat, Natriumcyanoborhydrid, gegebenenfalls in Kombination mit einem Edelmetallkatalysator) oder katalytisch aktivierter Wasserstoff.

Die Reduktion wird vorzugsweise in einem Lösungsmittel wie beispielsweise Methanol oder Ethanol ausgeführt bei Raumtemperatur oder gegebenenfalls unter erhöhter Temperatur bis zur Rückflußtemperatur des Reaktionsgemisches.

Ein Teil der Verbindungen der allgemeinen Formel I (unten dargestellt als Formel Ib) kann durch Reduktion einer entsprechenden Verbindung der allgemeinen Formel V hergestellt werden,

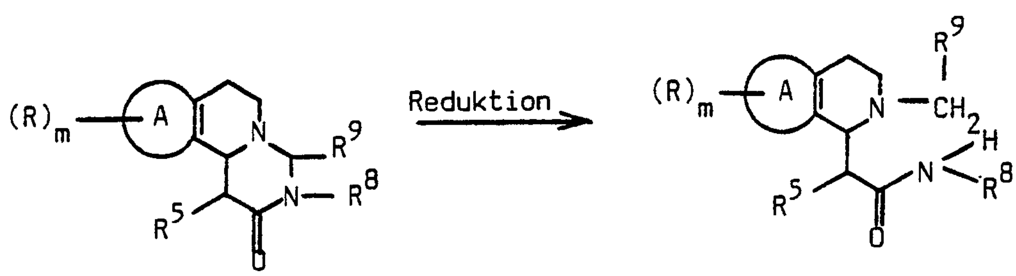


V Ib

worin

A einen Benzo-, Thieno- oder Indolorest bedeutet;

R Wasserstoff, $(C_1$-$C_4)$Alkyl, Halogen (F, Cl, Br, J), Hydroxy, $(C_1$-$C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;

m 1, 2 oder 3 bedeutet, wenn A ein Benzo- oder Indolorest ist, und 1 oder 2 bedeutet, wenn A ein Thienorest ist;

$R^9$ Wasserstoff oder $(C_1$-$C_3)$Alkyl bedeutet;

$R^5$ Wasserstoff, $(C_1$-$C_{10})$Alkyl, Phenyl, Phenyl$(C_1$-$C_5)$alkyl, $(C_1$-$C_4)$Alkoxy oder -NHCOX (worin X $(C_1$-$C_5)$-Alkyl ist) bedeutet;

$R^8$

(a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeutet, wobei das Alkyl

substituiert sein kann durch
Hydroxy,
$(C_1-C_4)$Alkoxy,
Di$(C_1-C_4)$Alkylamino,
Furyl,
Pyridyl,
Pyrrolidinyl,
Morpholino,
Indolyl,
Nitrilo,
Thienyl,
Phenyl oder Phenyl, das ein oder mehrfach durch Hydroxy, Methoxy oder Fluor substituiert ist;

(e) Phenyl, Fluorophenyl, Pyridyl oder N-Benzylpiperidyl; bedeutet.

Die Reduktion wird mit $NaBH_4$ in Eisessig, eventuell unter Zugabe eines Lösungsmittels (in Anlehnung an C. Djerassi, H.J. Monteiro, A. Walser, L.H. Durham J. Am. Chem Soc. 88, (1966) 1792) ausgeführt. Dazu wird eine Verbindung der allgemeinen Formel V in Eisessig gelöst und unter Kühlen und Rühren portionsweise mit einem Überschuß an Natriumboranat versetzt.

Die Reaktionsdauer wird sehr weitgehend von der Länge des Restes $R^5$ bestimmt und beträgt bei Raumtemperatur zwischen 1 und 15 Stunden. Die Reaktionszeiten nehmen in der Reihenfolge
$R^5 = H < CH_3 < C_2H_5 < C_4H_9 < C_5H_{11}$ ....
zu. Von $R^8$ werden sie nicht oder nur in unwesentlichem Maße beeinflußt.

Die Reaktionstemperatur ist weitgehend unkritisch und kann zwischen 5° C und der Siedetemperatur des Reaktionsgemisches liegen. Aus Sicherheitsgründen wird die Umsetzung unter Eiskühlung in einem Temperaturbereich zwischen 5° C und Raumtemperatur vorgenommen.

Als Lösungsmittel können neben Eisessig, der gleichzeitig als Reaktant auftritt, Gemische aus Eisessig und einem geeigneten, inerten Lösungsmittel Verwendung finden, bespielsweise THF, Dioxan, Ethanol etc.. Bevorzugt wird die Umsetzung in Eisessig.

Bevorzugte Ausführung der Reduktion:

Die Ausgangsverbindungen (V) werden in Eisessig gelöst. Unter Rühren und Kühlen auf 5° C wird das feinpulverisierte $NaBH_4$ (4 - 10facher Überschuß) portionsweise eingetragen. Nach beendeter Reaktion wird vorsichtig mit Wasser versetzt mit verdünnter NaOH alkalisch gestellt, mit $CH_2Cl_2$ extrahiert. Die organische Phase wird mit Wasser gewaschen, über $Na_2SO_4$ getrocknet und eingeengt. Der Rückstand wird, gegebenenfalls nach Reinigung über eine Kieselgelsäule (Eluens: $CH_2Cl_2$/MeOH 100:10) zur Kristallisation gebracht.

Die so erhaltenen Verbindungen der Formel I können einer oder mehreren der folgenden Nachbehandlungen unterworfen werden:

a) Eine Verbindung der allgemeinen Formel I, in der $R^6$ Hydroxy oder $(C_1-C_4)$Alkoxy ist, kann in das entsprechende Amid überführt werden, in dem $R^6$ die Gruppe $NR^7R^8$ ist. Dabei wird die Ausgangsverbindung, (gegebenenfalls nach vorherigem Schutz des basischen Ringstickstoffs und/oder Aktivierung der Carboxy- oder Estergruppe) mit dem entsprechenden Amin $NHR^7R^8$ (III) umgesetzt.

Als reaktionsfähige Carbonsäurederivate seien beispielsweise genannt: Säurehalogenide, Säureazide oder gemischte Säureanhydride (z.B. mit einer aromatischen oder aliphatischen Carbonsäure, Alkylkohlensäure oder Dialkylphosphorsäure usw.), ferner Säureamide (beispielsweise mit Imidazol, 4-substituiertem Imidazol, Dimethylpyrazol, Triazol oder Tetrazol usw.) oder aktive Ester (wie z.B. Cyanmethyl-, Methoxymethyl-, Vinyl-, Propargyl- oder P-Nitrophenylester usw.) oder Ester mit Dimethylhydroxylamin, 1-Hydroxysuccinimid, Dicyclohexylharnstoff usw., bevorzugt werden die aktiven Imidazolide.

Die Amidbildung wird in der Regel in einem inerten Lösungsmittel wie Dioxan, Acetonitril, Dimethylformamid oder einem Gemisch eines oder mehrerer dieser Lösungsmittel, gegebenenfalls in Gegenwart einer organischen oder anorganischen Base als Säurefänger, durchgeführt. Es ist aber auch möglich, die Reaktion ohne Lösungsmittel mit dem Amin im Überschuß durchzuführen.

Die Reaktionstemperatur kann, je nach eingesetzten Ausgangsstoffen, in weiten Grenzen variieren und zwischen etwa 0° C und der Siedetemperatur des Reaktionsgemisches liegen.

b) Eine Verbindung der allgemeinen Formel I, in der $R^4$ Wasserstoff ist, kann durch N-Alkylierung in die entsprechende Verbindung der Formel I überführt werden, in der $R^4$ $(C_1-C_4)$Alkyl ist.

Für die N-Alkylierung eignen sich im Prinzip alle bekannten Alkylierungsmittel, soweit sie eine ausreichende

Reaktivität besitzen, z.B. aktive Alkylester, wie Dialkylsulfat, Toluolsulfonsäurealkylester oder Fluorsulfonsäurealkylester oder Alkylhalogenide, wie Alkylbromide oder Alkyliodide. Die Reaktion erfolgt bei Temperaturen bis zum Siedepunkt des Reaktionsgemisches. Alkyl steht hier für $(C_1-C_4)$Alkyl,

Die Aminoalkylierung kann auch nach Leuckart-Wallach (Ber. dtsch. Chem. Ges. 18, (1985), 2341) oder Eschweiler-Clarke (Teilheimer 2, (1948) Nr. 352; 4 (1950) Nr. 378) ausgeführt werden. Im allgemeinen wird die Substanz mit einer ca. 30 %igen Aldehydlösung, bevorzugt Formalinlösung in Gegenwart von Ameisensäure bei Rückflußtemperatur behandelt. Die Zeitdauer variiert zwischen 3 und 18 Stunden. Diese Umsetzung ist besonders für Verbindungen (I) geeignet, in denen $R^6$ nicht $NH_2$ oder $NHR^8$ ist.

c) Eine Verbindung der Formel I, in der $R^6$ Hydroxy ist, kann in den entsprechenden Ester ($R^6$ = $(C_1-C_4)$Alkoxy) überführt werden.

d) Eine Verbindung der Formel I, in der $R^6$ $(C_1-C_4)$Alkoxy oder $NR^7R^8$ ist, kann zu der entsprechenden freien Säure hydrolysiert werden.

e) Die Diastereomerentrennung gelingt zum Teil spontan durch Kristallisation oder durch Anwendung der üblichen Methoden der Racemattrennung, z.B. Säulenchromatographie.

f) Die Überführung der freien Base der allgemeinen Formel I in ihre Säureadditionssalze erfolgt in an sich bekannter Weise.

Zur Salzbildung geeignete Säuren sind beispielsweise Salzsäure, Bromwasserstoffsäure, Schwefelsäure, Phosphorsäure, Salpetersäure, Essigsäure, Propionsäure, Buttersäure, Oxalsäure, Malonsäure, Bernsteinsäure, Maleinsäure, Fumarsäure, Milchsäure, Weinsäure, Zitronensäure, Äpfelsäure, Benzoesäure, Zimtsäure, Ascorbinsäure oder Methansulfonsäure.

Die Verbindungen der Formel II können nach an sich bekannten Verfahren hergestellt werden, vorzugsweise nach der in der deutschen Patentanmeldung P 37 18 570.5 beschriebenen Methode.

In Gegenwart eines Kondensationsmittels kann ein Malonsäurediamid der allgemeinen Formel IV,

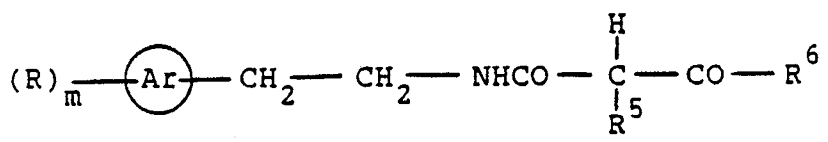
(IV)

in der R, m, $R^5$ und $R^6$ wie oben definiert sind und Ar Phenyl, 2- oder 3-Indolyl oder 2- oder 3-Thienyl bedeutet,zu den entsprechenden Verbindungen II und II′, cyclisiert werden.

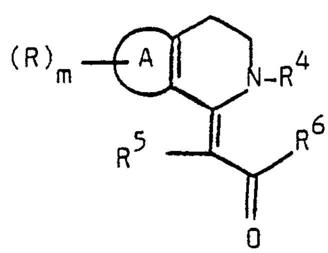
II

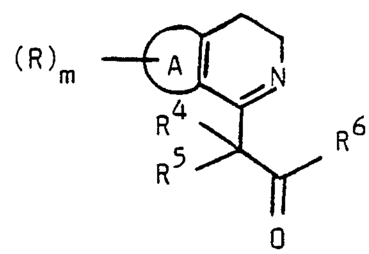
II'

Hierbei sind A, m, R, $R^5$ und $R^6$ wie oben für die Verbindungen IV definiert, $R^4$ ist Wasserstoff oder $(C_1-C_4)$Alkyl.

Auf die Ausführung des Verfahrens wird weiter unten näher eingegangen. Wird die Reaktion z.B. mit einem Gemisch von Phosphorpentoxid und $(C_1-C_4)$Alkylsulfonsäure ausgeführt, so erhält man neben den entsprechenden Verbindungen II und II′, worin $R^4$ Wasserstoff ist, auch die analogen Verbindungen II und II′, worin $R^4$ $(C_1-C_4)$Alkyl ist.

Als Kondensationsmittel für dieses Verfahren eignen sich starke Lewis-Säuren, wie z.B. Phosphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phorphoroxychlorid und Phosphorpentachlorid, oder ein Gemisch von Phosphorpentoxid und $(C_1-C_4)$ Alkylsulfonsäure z.B. mit einem $P_2O_5$-Anteil von ca. 10 Gewichtsprozenten.

Wird eine Verbindung IV in Gegenwart des Gemisches von Phosphorpentoxid und $(C_1-C_4)$-Alkylsulfonsäure cyclisiert, so erhält man, wie oben erwähnt worden ist, neben den entsprechenden

Verbindungen II und II′, in denen $R^4$ Wasserstoff ist, auch die analogen Verbindungen II und II′, in denen $R^4$ $(C_1-C_4)$Alkyl ist. Vorzugsweise wird diese Verfahrensvariante mit Methansulfonsäure ausgeführt.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbezole (z.B. Toluol, Xylol), Chlorbenzole, Chloroform, Acetonitril, Dekalin. Eine bevorzugte Variante des Verfahrens besteht darin, das Kondensationsmittel, beispielsweise Phosphoroxychlorid oder ein $(C_1-C_4)$-Alkylsulfonsäure-/Phosphorpentoxid-Gemisch, ohne Zusatz von Lösungsmittel zu verwenden.

Bevorzugt erfolgt die Cyclisierung mit Phosphoroxychlorid oder in schwierigen Fällen mit einem Gemisch von Phosphorpentoxid und $(C_1-C_4)$Alkylsulfonsäure (bevorzugt Methansulfonsäure).
Die Umsetzung kann innerhalb eines großen Temperaturbereichs, vorzugsweise unter Erwärmen oder Erhitzen auf 50° C bis etwa den Siedepunkt des Reaktionsgemisches, durchgeführt werden.

Die erforderliche Reaktionsdauer liegt je nach Ausgangsverbindung IV zwischen 2 und 15 Stunden.

Die Tautomeren der allgemeinen Formeln II und II′, worin $R^4$ Wasserstoff ist, können nach bekannten Vefahren getrennt werden, wie z.B. durch Säulenchromatographie.

Die Verbindungen der allgemeinen Formel II beziehungsweise II′, in denen A ein Indolorest ist, R, m, $R^4$, $R^5$ und $R^6$ wie oben für die Verbindungen der allgemeinen Formel I definiert sind, sind neue Verbindungen, ausgenommen diejenigen,
worin
m eins ist,
R Wasserstoff, $(C_1-C_4)$Alkyl, Halogen oder $(C_1-C_4)$Alkoxy bedeutet,
$R^4$ Wasserstoff oder $(C_1-C_4)$Alkyl bedeutet,
$R^5$ Wasserstoff bedeutet,
$R^6$ $(C_1-C_4)$Alkoxy oder eine $-NHR^8$-Gruppe bedeutet,
worin
$R^8$ Wasserstoff oder $(C_1-C_4)$-Alkyl bedeutet, wobei das Alkyl substituiert sein kann durch Phenyl.

Die Erfindung betrifft ferner neue Verbindungen der allgemeinen Formel V,

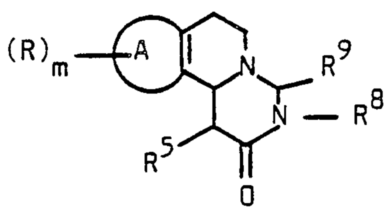

V

worin
A einen Benzo-, Thieno- oder Indolorest bedeutet;
R Wasserstoff, $(C_1-C_4)$Alkyl, Halogen (F, Cl, Br, J), Hydroxy, $(C_1-C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;
m 1, 2 oder 3 bedeutet, wenn A ein Benzo- oder Indolorest ist, und 1 oder 2 bedeutet, wenn A ein Thienorest ist;
$R^9$ Wasserstoff oder $(C_1-C_3)$Alkyl bedeutet;
$R^5$ Wasserstoff, $(C_1-C_{10})$Alkyl, Phenyl, Phenyl$(C_1-C_5)$alkyl, $(C_1-C_4)$Alkoxy oder -NHCOX (worin X $(C_1-C_5)$-Alkyl ist) bedeutet;
$R^8$

(a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeutet, wobei das Alkyl substituiert sein kann durch
Hydroxy,
$(C_1-C_4)$Alkoxy,
Di$(C_1-C_4)$Alkylamino,
Furyl,
Pyridyl,

Pyrrolidinyl,
Morpholino,
Indolyl,
Nitrilo,
Thienyl,
Phenyl oder Phenyl, das ein oder mehrfach durch Hydroxy, Methoxy oder Fluor substituiert ist;

(e) Phenyl, Fluorophenyl, Pyridyl oder N-Benzylpiperidyl; bedeutet und deren Salze mit einer anorganischen oder organischen Säure. Diese Verbindungen sind Ausgangsverbindungen für die entsprechenden Verbindungen der allgemeinen Formel I, worin $R^4$ die Gruppe $-CR^9H_2$ ist und $R^6$ die Gruppe $-NHR^8$. Auch diese Verbindungen zeigen kardioprotektive Wirkung.

Ferner betrifft die Erfindung eine pharmazeutische Zubereitung enthaltend eine Verbindung der Formel V, die wie oben definiert ist, oder deren pharmazeutisch annehmbares Salz mit einer anorganischen oder organischen Säure.

Die Verbindungen der allgemeinen Formel V können hergestellt werden, indem man eine entsprechende Verbindung der allgemeinen Formel I, worin $R^4$ Wasserstoff ist und $R^6$ die Gruppe $-NHR^8$ bedeutet und R, m, $R^5$ und $R^8$ wie in der gewünschten Verbindung der Formel V definiert sind (diese Verbindung entspricht der allgemeinen Formel I'), mit einem Aldehyd der allgemeinen Formel $R^9CHO$ (VI) umsetzt:

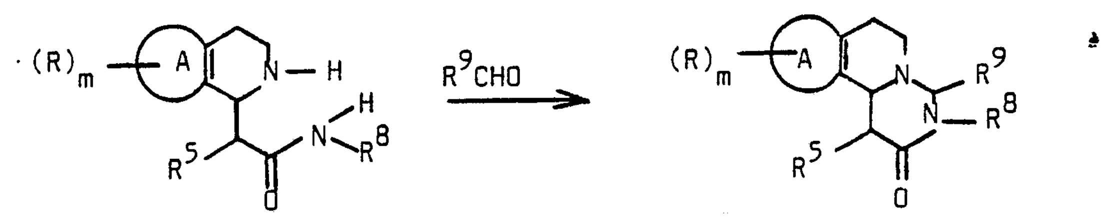


I' V

$R^9$ bedeutet Wasserstoff oder $(C_1-C_3)$Alkyl. Die Umsetzung erfolgt zweckmäßig mit einer 30 %igen Aldehydrlösung bei erhöhter Temperatur. Vorzugsweise wird die Umsetzung mit Formalinlösung in Gegenwart von Ameisensäure bei Rückflußtemperatur ausgeführt.

Die durch die allgemeinen Formeln I und V beschriebenen Verbindungen besitzen, wie eingangs erwähnt, sowohl als Basen als auch in Form ihrer Salze wertvolle therapeutische Eigenschaften. Insbesondere weisen diese Substanzen eine deutliche cardioprotektive Wirkung auf, die wie folgt bestimmt wurde:

Bekanntlich ist der myocardiale $Ca^2$-Gehalt ein Maß für die hypoxische bzw. durch toxische Katecholamindosen hervorgerufene Herzschädigung (Higgins et al., Mol. Cell. Cardial. 10, 427-438, 1984; Nakanishi et al., Am. J. Physiol. 242. 437-449, 1982; Fleckenstein, A. Vorträge der Erlanger Physiol. Tagung 1970, Edit. Keidel, Springer Verlag Berlin, Heidelberg, New York, 1971). Umgekehrt ist die Inhibition der hypoxischen oder Isoprenalin bedingten myocardialen Calciumaufnahme ein Maß für die cardioprotektive Effektivität von Calciumantagnosten (Fleckenstein, s.o.). von Calmodulininhibitoren (Higgins) und anderen Pharmaka, z.B. Betaadrenolytika (Anrdts, Arzneimittelforschung 25, 1279-1284, 1975). Die cardioprotektive Wirkung wurde an wachen Ratten nach subcutaner oder peroraler Wirkstoffgabe anhand der von Arndts (s.o.) beschriebenen Methode festgestellt und die Wirkungsstärke der Testsubstanzen als $H_{50}$-Wert angegeben. Dieser Wert entspricht der Dosis, die durch eine Gabe von 30 mg/kg s.c. Isoprenalin bedingte myocardiale Radiocalciumaufnahme zu 50 % hemmt.

Hier erweisen sich die untersuchten Verbindungen als bis zu fünfmal wirksamer als das bekannte Handelsprodukt Propranolol.

In vitro-Untersuchungen an der glatten Muskulatur (Aortenstreifen) haben ergeben, daß es sich bei den erfindungsgemäßen Verbindungen am Calciumantagonisten mit einem neuen Wirkungsmechanismus handelt:

Calciumantagonisten hemmen den transmembranären Calciumioneneinstrom in die Zelle. Diese Hemmung betrifft den spannungsabhängigen (langsamen) Calciumkanal in der Zellmembran. Die Bestimmung transmembranärer Calciumionenströme an Gewebestreifen unter Kaliumdepolarisation nach der von van Breemen beschriebenen Methode weist Calciumantagonisten eindeutig nach (van Breemen et al., Chest. 78,

157, S - 165; 1980; van Breemen et al., Am. J. Cardiol. 49, 507-510, 1982; Casteels et al. Pflügers Arch. 392, 139-145, 1981; Deth und van Breemen, J. Membrane Biol. 30, 363-380, 1977).

Untersuchung am isolierten Rattenherzen

Wird ein, längere Zeit unter ischämischen Bedingungen gehaltenes, isoliertes Herz wieder normal perfundiert, so tritt keine sofortige Normalisierung der Herzfunktion ein. Es findet sich vielmehr eine vorübergehende Periode, die durch Kontraktur und Arrhytmien gekennzeichnet ist. Diese Arrhythmiephase ist durch Veränderungen von Funktion und Struktur der Myocardzelle mit intrazellulärer Calciumüberladung bedingt (Hess u. Manson, J. Mol. Cell. Cardiol. 16, 969, 1984). Cardioprotektiv wirksame Verbindungen wie Verapamil und Diltiazem verringern die Calciumüberladung und verbessern das Kontraktionsverhalten bei Reperfusion (Watts et al, Am. J. Physiol. 238, H 909, 1980; Meno et al, Am. J. Physiol. 247, H 380, 1984), Die cardioprotektive Wirkung wird an isolierten Rattenherzen unter Ischämie und anschließender Reperfusion untersucht.

Methodik I:

Isolierte Herzen werden durch weitgehende Reduzierung des Coronarflusses (über 95 %) einem ausprägten Sauerstoff-Mangel ausgesetzt. Infolge Störungen des Herzstoffwechsels findet sich ein rasch eintretender Stillstand der Herztätigkeit, nach Ende der Coronarflußreduzierung setzt die Herztätigkeit nur verzögert und unregelmäßig wieder ein, eine regelmäßige Aktion wird erst nach 10 - 15 min beobachtet. Cardioprotektive Verbindungen, während der Periode der Flußreduzierung dem Perfusionsmedium zugesetzt, verkürzen die Periode unregelmäßiger Herztätigkeit.

| Verbindung | konz. [µ/ml] | Verkürzung der Arrhythmiephase 10-15 min auf |
|---|---|---|
| A | 13,2-26,6 | 6,2-10 |
| B | 6,6-13,3 | 4,0-4,8 |
| C | 6,6 | 2,3 |
| Alinidin | 13,3-33,3 | 3,25-4,15 |
| Propranolol | 6,6 | 2,6 min |
| F | 6,6 | 3,75 min |

Methodik II:

Wird der Coronarfluß isolierter Herzen um 75 % gedrosselt, so reagiert das Herz zunächst mit einer Abnahme der Kontraktionsamplitude, ein Herzstillstand tritt erst innerhalb einer Stunde ein. Cardioprotektive Substanzen, während der Periode der Flußreduzierung dem Perfusionsmedium zugesetzt, verhindern den Abfall der Kontraktionsamplitude.

| | Verbindung | Benötige Konzentrationen |
|---|---|---|
| | D | 3,0 µg/ml, |
| | E | 1,0 µg/ml, |
| Vergleich: | Alinidin | 8,3 µg/ml. |

Verbindungen A bis F :

A

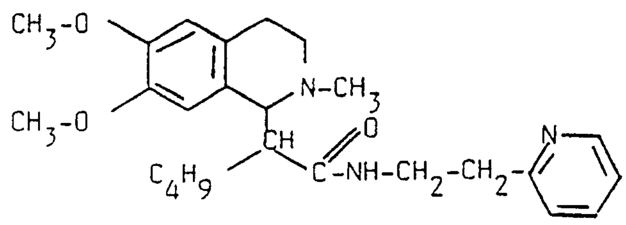

B

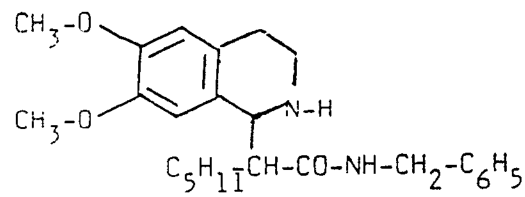

C

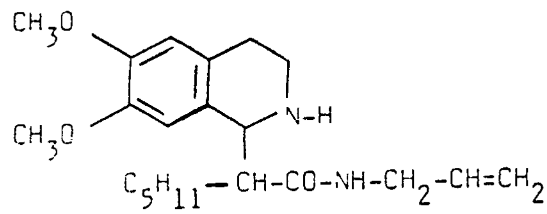

D

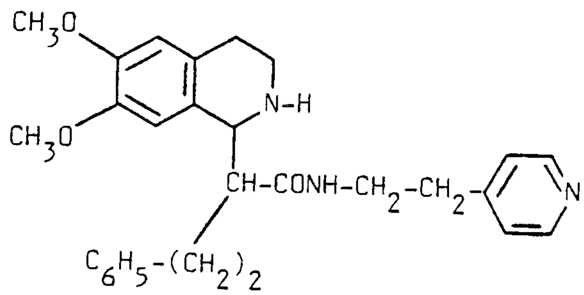

E

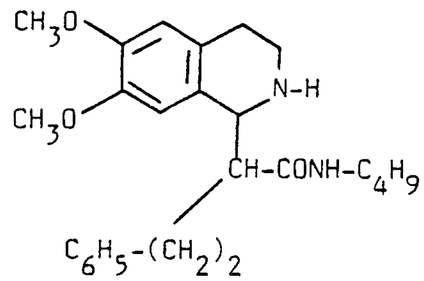

F

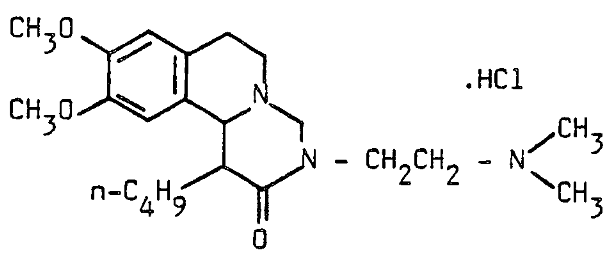

Aufgrund dieser Befunde können die Verbindungen der allgemeinen Formel I beziehungsweise ihre Säuresalze als Wirkstoffe für Arzneimittel gegen die koronare Herzkrankheit Verwendung finden.

Ein weiterer Gegenstand der Erfindung sind Arzneimittel, welche die Substanzen der allgemeinen Formeln I und V sowie ihre Salze mit anorganischen oder organischen Säuren enthalten. Die Arzneimittel sind für orale oder parenterale Verabreichung geeinget. Als Arzneimittelformen dienen vorwiegend Tabletten, Dragees, Ampullen und Saftzubereitungen. Die Einzeldosis zu diesen Arzneiformen beträgt zwischen 1,0 und 200 mg, vorzugsweise 20 und 50 mg pro 75 kg Körpergewicht. Je nach Schwere des Falles sind täglich im allgemeinen 1 bis 3 Einzeldosen zu verabreichen.

Beispiel 1

2-(1,2,3,4-Tetrahydro-6-methylmerkapto-1-isochinolinyl)-N-(1-pentyl)-hexanamid.

2,5 g 2-(3,4-Dihydro-6-methylmerkapto-1-isochinolinyl)-N-(1-pentyl)-hexanamid werden in 100 ml Methanol portionsweise mit 0,5 g $NaBH_4$ versetzt und 3 Stunden bei Raumtemperatur gerührt. Nach beendeter Reaktion wird überschüssiges $NaBH_4$ zersetzt, das Lösungsmittel im Vakuum abgezogen, der Rückstand zwischen $CH_2Cl_2$ und Wasser verteilt. Nach dem Trocknen der organischen Phase über wasserfreiem Natriumsulfat wird eingedampft und der Rückstand aus Essigester/Petrolether kristallisiert.
Fp. 95 °C

Beispiel 2

2-(1,2,3,4-Tetrahydro-6-methylmerkapto-1-isochinolinyl)-hexansäureethylester-hydrochlorid.

20 g 2-(3,4-Dihydro-6-methylmerkapto-1-isochinolinyl)-hexansäureethylester werden in 200 ml siedendem Ethanol portionsweise mit 5,2 g festem $NaBH_4$ versetzt und 2 Stunden bei Siedetemperatur gerührt. Nach der Aufarbeitung des Reaktionsgemisches wird das Hydrochlorid gebildet und aus Ethanol/Ether kristallisiert.
Fp. 123 °C

Beispiel 3

2-(1,2,3,4-Tetrahydro-6,7-dimethoxy-1-isochinolinyl)-hexansäureethylester-hydrochlorid

60 g 2-(3,4-Dihydro-6,7-dimethoxy-1-isochinolinyl)-hexansäureethylester werden in 600 ml Ethanol unter Zusatz von 17 g konzentrierter HCl in Gegenwart von 6 g $PtO_2$ bei 20 °C und 5 bar mit Wasserstoff katalytisch hydriert. Nach dem Entfernen des Katalysators wird die Reaktionslösung eingeengt, und das Produkt durch Zugabe von Ether zur Kristallisation gebracht.
Fp. 141-145 °C

Beispiel 4

2-(1,2,3,4-Tetrahydro-6,7-dimethoxy-2-N-methyl-1-isochinolinyl)-hexansäureethylester-hydrochlorid

Ein Gemisch aus 12 g 2-(1,2,3,4-Tetrahydro-6,7-dimethoxy-1-isochinolinyl)-hexansäureethylester, 33 g Formalin (30 %) und 16 g Ameisensäure (98 %) wird 1 Stunde zum Sieden erhitzt. Nach dem Eindampfen des Reaktionsgemisches wird mit halbgesättigter Sodalösung alkalisch gestellt, mit $CH_2Cl_2$ extrahiert, die organische Phase mit Wasser gewaschen, über $Na_2SO_4$ getrocknet und im Vakuum eingedampft. Das Reaktionsprodukt wird an $Al_2O_3$ Aktivitätsstufe III (Eluens $CCl_4$) gereinigt und in das Hydrochlorid überführt.
Fp. 156-157 °C (Ethanol/Ether)

Beispiel 5

2-(1,2,3,4-Tetrahydro-6,7-dimethoxy-2-N-methyl-1-isochinolinyl)-hexansäure-hydrochlorid

15,5 g 2-(1,2,3,4-Tetrahydro-6,7-dimethoxy-1-isochinolinyl)-hexansäureethylester werden in 100 ml konz. Salzsäure 1,5 Stunden zum Sieden erhitzt. Nach beendeter Reaktion wird im Vakuum weitgehend eingeengt und ein Diastereomer durch Zugabe von Aceton zur Kristallisation gebracht (Fp. 180-183 °C), das zweite Diastereomer verbleibt in der Mutterlauge.

Beispiel 6

2-(1,2,3,4-Tetrahydro-6-methylmerkapto-1-isochinolinyl)-N-(1-pentyl)-hexanamid

3 g 2-(1,2,3,4-Tetrahydro-6-methylmerkapto-1-isochinolinyl)-hexansäureethylester werden in 50 ml n-Pentylamin ca. 14 Stunden zum Sieden erhitzt. Nach beendeter Umsetzung (DC-Kontrolle) wird überschüssiges Amin im Vakuum entfernt, der Rückstand in $CH_2Cl_2$ aufgenommen, mehrmals mit Wasser ausgeschüttelt, die organische Phase über wasserfreiem $Na_2SO_4$ getrocknet, eingedampft und der Rückstand an Kieselgel (Eluens $CH_2Cl_2$/MeOH = 100:3) gereinigt.
Fp. 95 °C

Beispiel 7

2-(1,2,3,4-Tetrahydro-6,7-dimethoxy-2-N-methyl-1-isochinolinyl)-hexan-N-[2-(2,4-dimethoxyphenyl)-ethyl]-amid

2,58 g 2-(1,2,3,4-Tetra-hydro-6,7-dimethoxy-2-N-methyl-1-isochinolinyl)-hexan-säure werden in 50 ml wasserfreiem DMF mit 3,2 g N,N′-carbonyldiimidazol 4 Stunden bei Raumtemperatur gerührt. Danach wird mit 3 ml 3,4-Dimethoxyphenylethyl- amin versetzt und nach 15-stündigem Stehen bei Raumtemperatur aufgearbeitet. Diese Umsetzung verläuft ohne Strukturänderung. Wird als Ausgangsmaterial ein Isomeres eingesetzt, so erhält man das entsprechende Isomere der Endverbindung. Diastereomer 1: Fp. 94-96° C (Essigester/Ligroin) Diastereomer 2: Fp. 75-78° C (Essigester/Ligroin)

Beispiel 8

2-(1,2,3,4-Tetrahydro-6,7-dimethoxy-1-isochinolinyl)-hexan-N-(2-phenylethyl)-amid-hydrochlorid

a) 2-[1,2,3,4-Tetrahydro-6,7-dimethoxy-2-N-(9-fluorenylmethyloxycarbonyl)-1-isochinolinyl]-hexansäure.
In ein Gemisch aus 10,8 g 2-(1,2,3,4-Tetrahydro-6,7-dimethoxy-1-isochinolinyl)-hexansäure, 70 ml wäßriger Sodalösung (10%) und 35 ml Dioxan wird bei 0° C im Verlauf von 30 min eine Lösung von 9,1 g 9-Fluorenylmethyloxycarbonylchlorid in 55 ml Dioxan zugetropft. Nach 1-stündigem Rühren bei 0° C läßt man 15 Stunden bei Raumtemperatur stehen, gießt auf 1000 ml Eiswasser und extrahiert 3 mal mit je 100 ml Ether. Die wäßrige Phase wird auf pH = 2 gebracht, der feste weiße Niederschlag abgesaugt und nach Lösen in $CHCl_3$/MeOH (1:1) durch Zugabe von Petrolether kristallisiert.
Fp. 169-172° C.

b) 2-[1,2,3,4-Tetrahydro-6,7-dimethoxy-2-N-(9-fluorenylmethyloxycarbonyl)-1-isochinolinyl]-hexan-N-(2-phenylethyl)-amid
2,65 g 2-[1,2,3,4-Tetrahydro-6,7-dimethoxy-2-N-(9-fluorenyl-methyloxycarbonyl)-1-isochinolinyl]-hexansäure werden in 15 ml wasserfreiem DMF mit 1 g N,N′-carbonyldiimidazol zur Reaktion gebracht. Nach 1 Stunde wird mit einer äquivalenten Menge 2-Phenylethylamin versetzt; nach weiteren 15 Stunden bei Raumtemperatur wird aufgearbeitet, über Kieselgel (Eluens $CHCl_2$/MeOH = 100:2) gereinigt und aus Essigester/Petrolether kristallisiert. Fp. 168-170° C.

c) 1,5 g 2-[1,2,3,4-Tetrahydro-6,7-dimethoxy-2-N-(9-fluorenylmethyloxycarbonyl)-1-isochinolinyl]-hexan-N-(2-phenylethyl)-amid werden in 30 ml eines Gemisches aus Trifluoressigsäure und Anisol (1%) 15 Stunden bei Raumtemperatur gerührt. Nach dem Entfernen des Solvens wird der Rückstand zwischen $CH_2Cl_2$ und einer 10%igen Natriumcarbonatlösung verteilt, die organische Phase mit Wasser gewaschen, über $Na_2SO_4$ getrocknet, eingeengt, das Hydrochlorid gebildet und aus Ethanol/Ether kristallisiert.
Fp. 154-156° C.

Beispiel 9

2-(1,2,3,4-Tetrahydro-6,7-dimethoxy-2-methyl-1-isochinolinyl-buttersäureethylester-hydrochlorid

a) 2-(3,4-Dihydro-6,7-dimethoxy-1-isochinolinyl)-buttersäure-ethylester-methojodid.
0,35 g 2-(3,4-Dihydro-6,7-dimethoxy-1-isochinolinyl)-buttersäureethylester und 1 ml Methyljodid werden in 10 ml eines Ethanol/Nitromethan-Gemisches (1:1) 1,5 h auf 60° C erhitzt. Nach vollständiger Umsetzung (DC-Kontrolle) wird im Vakuum eingedampft und der Rückstand aus Ethanol/Petrolether kristallisiert.
Fp. 183-185° C.

b) 0,5 g 2-(3,4-Dihydro-6,7-dimethoxy-1-isochinolinyl)-buttersäureethylester-methojodid werden in 5 ml Ethanol bei Raumtemperatur mit 0,1 g $NaBH_4$ 3 Stunden gerührt. Nach beendeter Reaktion (DC-Kontrolle) wird wie üblich aufgearbeitet und das Hydrochlorid gebildet.
Fp. 164-166° C

Beispiel 10

2-(1H-1,2,3,4-Tetrahydropyrido[3,4-b]indol-1-yl)-N-(2-morpholinoethyl)-acetamid-hydrochlorid

a) 2-(1H-3,4-Dihydropyrido[3,4-b]indol-1-yl)-N-(2-morpholinoethyl)-acetamid

15 g 2-(3-Indolyl)ethylaminocarbonylessigsäure-N-(2-morpholinoethyl)amid und 45 ml $POCl_3$ werden in 250 ml eines Acetonitril-Benzol-Gemisches (1:1) 5 Stunden auf 40-45 °C erwärmt. Nach dem Entfernen des Lösungsmittels und von überschüssigem $POCl_3$ wird der Rückstand zwischen Eiswasser und Chloroform verteilt, über $Al_2O_3$ (Eluens: $CH_2Cl_2$/MeOH = 100:3) gereinigt und aus Essigester/Äther unter Zugabe von Ligroin 55-75 °C kristallisiert.

b) Das so erhaltene Produkt wird analog Beispiel 1 reduziert, das Produkt isoliert und in das Hydrochlorid (Titelverbindung) überführt.

Fp. 262-265 °C

Nach den beschriebenen Verfahren beziehungsweise den obigen Beispielen können beispielsweise auch die in den folgenden Tabellen zusammengefaßten Verbindungen hergestellt werden.

Tabelle 1:
==========

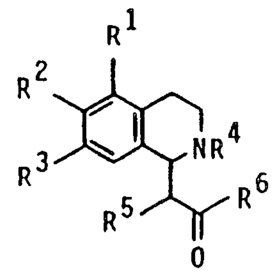

| Nr. | $R^1$ | $R^2$ | $R^3$ | $R^4$ | $R^5$ | $R^6$ | Fp | Salzform |
|---|---|---|---|---|---|---|---|---|
| 1 | H | $OCH_3$ | H | H | H | $NH(CH_2)_4CH_3$ | 130-134 | HCl |
| 2 | H | $OCH_3$ | OH | H | H | $OC_2H_5$ | 111-114 | BS |
| 3 | H | $OCH_3$ | $OCH_3$ | H | H | $NHCH_3$ | 221-225 | HCl |
| 4 | H | $OCH_3$ | $OCH_3$ | H | H | $NHCH_2CH_3$ | 137-140 | HCl |
| 5 | H | $OCH_3$ | $OCH_3$ | H | H | $NH-CH_2C{\equiv}CH$ | 209-213 | HCl |
| 6 | H | $OCH_3$ | $OCH_3$ | H | H | $NHCH_2CH=CH_2$ | 224-226 | BS |
| 7 | H | $OCH_3$ | $OCH_3$ | H | H | $NHCH(CH_3)_2$ | 116-118 | BS |
| 8 | H | $OCH_3$ | $OCH_3$ | H | H | 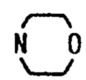 | 182-184 | HCl |
| 9 | H | $OCH_3$ | $OCH_3$ | $CH_3$ | H | $N(CH_3)_2$ | 92- 98 | BS |
| 10 | H | $OCH_3$ | $OCH_3$ | $CH_3$ | H | $N(C_2H_5)_2$ | 76- 78 | BS |
| 11 | H | $OCH_3$ | $OCH_3$ | $CH_3$ | H | 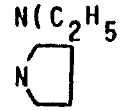 | 125-128 | BS |
| 12 | H | $OCH_3$ | $OCH_3$ | $CH_3$ | H | 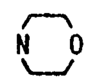 | 110-112 | BS |
| 13 | H | $OCH_3$ | $OCH_3$ | H | $CH_3$ | $OC_2H_5$ | 160-163 | HCl |

| Nr. | $R^1$ | $R^2$ | $R^3$ | $R^4$ | $R^5$ | $R^6$ | Fp | Salzform |
|---|---|---|---|---|---|---|---|---|
| 14 | H | $OCH_3$ | $OCH_3$ | H | $CH_3$ | $NHCH_2CH(CH_3)_2$ | 198-202 | HCl |
| 15 | H | $OCH_3$ | $OCH_3$ | H | $CH_3$ | $NHCH_2CH(CH_3)_2$ | 183-185 | HCl |
| 16 | H | $OCH_3$ | $OCH_3$ | H | $CH_3$ | $N((CH_2)_2CH_3)_2$ | 97-101 | HCl |
| 17 | H | $OCH_3$ | $OCH_3$ | H | $CH_3$ | $N((CH_2)_2CH_3)_2$ | 225-229 | HCl |
| 18 | H | $OCH_3$ | $OCH_3$ | H | $CH_3$ | $N((CH_2)_3CH_3)_2$ | 66- 74 | HCl |
| 19 | H | $OCH_3$ | $OCH_3$ | H | $CH_3$ | $N((CH_2)_3CH_3)_2$ | 201-203 | HCl |
| 20 | H | $OCH_3$ | $OCH_3$ | H | $CH_3$ | $NH(CH_2)_4CH_3$ | 202-205 | HCl |
| 21 | H | $OCH_3$ | $OCH_3$ | H | $CH_3$ | $NH-CH_2-$ 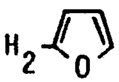 | 187-192 | HCl |
| 22 | H | $OCH_3$ | $OCH_3$ | H | $CH_3$ | $NH-CH_2-$ 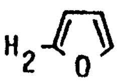 | 206-211 | HCl |
| 23 | H | $OCH_3$ | $OCH_3$ | H | $CH_3$ | $NH-(CH_2)_2-$ 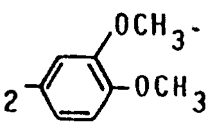 | 113-117 | BS |
| 24 | H | $OCH_3$ | $OCH_3$ | H | $CH_3$ | $NH-(CH_2)_2-$ 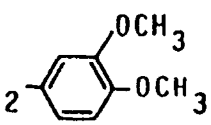 | 194-197 | HCl |
| 25 | H | $OCH_3$ | $OCH_3$ | H | $CH_3$ | $NH-(CH_2)_2-$ 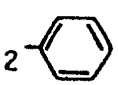 | 198-201 | HCl |
| 26 | H | $OCH_3$ | $OCH_3$ | H | $CH_3$ | $NH-(CH_2)_2-$ 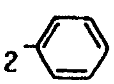 | 201-206 | HCl |

| Nr. | $R^1$ | $R^2$ | $R^3$ | $R^4$ | $R^5$ | $R^6$ | Fp | Salzform |
|---|---|---|---|---|---|---|---|---|
| 27 | H | $OCH_3$ | $OCH_3$ | H | $CH_3$ | $NH-(CH_2)_2-OCH_3$ | 168-172 | HCl |
| 28 | H | $OCH_3$ | $OCH_3$ | H | $CH_3$ | $NH-(CH_2)_2-$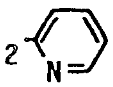 | 115-121 | HCl |
| 29 | H | $OCH_3$ | $OCH_3$ | H | $CH_3$ | $NH-(CH_2)_2-$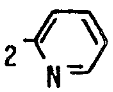 | 99-100 | BS |
| 30 | H | $OCH_3$ | $OCH_3$ | H | $CH_3$ | NH-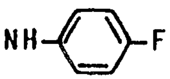 | 218-221 | HCl |
| 31 | H | $OCH_3$ | $OCH_3$ | H | $CH_3$ | $NHCH(CH_3)CH_2CH_2CH_2CH(CH_3)_2$ | 194-195 | HCl |
| 32 | H | $OCH_3$ | $OCH_3$ | H | $CH_3$ | $NH(CH_2)_3N(CH_3)_2$ | 81- 94 | HCl |
| 33 | H | $OCH_3$ | $OCH_3$ | H | $CH_3$ | $NH(CH_2)_2-$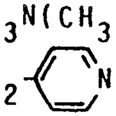 | 201-206 | HCl |
| 34 | H | $OCH_3$ | $OCH_3$ | $CH_3$ | $CH_3$ | $NH(CH_2)_2CH_3$ | 188-191 | HCl |
| 35 | H | $OCH_3$ | $OCH_3$ | $CH_3$ | $CH_3$ | $NH-(CH_2)_2OCH_3$ | 169-172 | HCl |
| 36 | H | $OCH_3$ | $OCH_3$ | $CH_3$ | $CH_3$ | $NH-CH_2-CH(CH_3)_2$ | 192-195 | HCl |
| 37 | H | $OCH_3$ | $OCH_3$ | $CH_3$ | $CH_3$ | $NH(CH_2)_2-$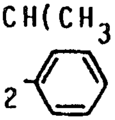 | 185-188 | HCl |
| 38 | H | $OCH_3$ | $OCH_3$ | $CH_3$ | $CH_3$ | $NH-(CH_2)_2-$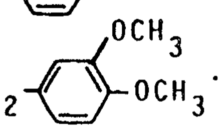 | 184-187 | HCl |
| 39 | H | $OCH_3$ | $OCH_3$ | $CH_3$ | $CH_3$ | NH-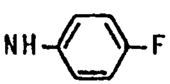 | 173-176 | HCl |
| 40 | H | $OCH_3$ | $OCH_3$ | H | $C_2H_5$ | $NHCH_2CH(CH_3)_2$ | 206-208 | HCl |
| 41 | H | $OCH_3$ | $OCH_3$ | H | $C_2H_5$ | $NH(CH_2)_4CH_3$ | 205-206 | HCl |

| Nr. | $R^1$ | $R^2$ | $R^3$ | $R^4$ | $R^5$ | $R^6$ | Fp | Salzform |
|---|---|---|---|---|---|---|---|---|
| 42 | H | $OCH_3$ | $OCH_3$ | H | $C_2H_5$ | $NH(CH_2)_2OCH_3$ | 194-195 | HCl |
| 43 | H | $OCH_3$ | $OCH_3$ | H | $C_2H_5$ | $N((CH_2)_2CH_3)_2$ | 187-190 | HCl |
| 44 | H | $OCH_3$ | $OCH_3$ | H | $C_2H_5$ | $N((CH_2)_3CH_3)_2$ | 189-191 | HCl |
| 45 | H | $OCH_3$ | $OCH_3$ | H | $C_2H_5$ | $NH-CH_2-$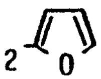 | 202-206 | HCl |
| 46 | H | $OCH_3$ | $OCH_3$ | H | $C_2H_5$ | NH-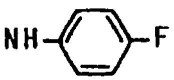 | 167-201 | HCl |
| 47 | H | $OCH_3$ | $OCH_3$ | H | $C_2H_5$ | $NHCH(CH_3)CH_2CH_2CH_2CH(CH_3)_2$ | 189-198 | HCl |
| 48 | H | $OCH_3$ | $OCH_3$ | H | $C_2H_5$ | $NH-(CH_2)_2-$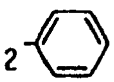 | 212-214 | HCl |
| 49 | H | $OCH_3$ | $OCH_3$ | H | $C_2H_5$ | $NH-(CH_2)_2-$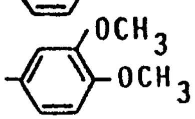 | 197-201 | HCl |
| 50 | H | $OCH_3$ | $OCH_3$ | H | $C_2H_5$ | $NH-(CH_2)_2-$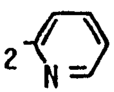 | 165-179 | HCl |
| 51 | H | $OCH_3$ | $OCH_3$ | H | $C_2H_5$ | $NH(CH_2)_2-$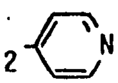 | 138-165(z) | HCl |
| 52 | H | $OCH_3$ | $OCH_3$ | H | $C_2H_5$ | $NH(CH_2)_3N(CH_3)_2$ | 129-146(z) | HCl |
| 53 | H | $OCH_3$ | $OCH_3$ | H | $C_2H_5$ | $OC_2H_5$ | 201-206 | HCl |
| 54 | H | $OCH_3$ | $OCH_3$ | $CH_3$ | $C_2H_5$ | $N((CH_2)_2CH_3)_2$ | 184-185 | HCl |
| 55 | H | $OCH_3$ | $OCH_3$ | $CH_3$ | $C_2H_5$ | $N((CH_2)_3CH_3)_2$ | 184-185 | HCl |
| 56 | H | $OCH_3$ | $OCH_3$ | $CH_3$ | $C_2H_5$ | $NH(CH_2)_2-$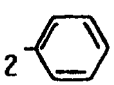 | 145-147 | HCl |

| Nr. | $R^1$ | $R^2$ | $R^3$ | $R^4$ | $R^5$ | $R^6$ | Fp | Salzform |
|---|---|---|---|---|---|---|---|---|
| 57 | H | $OCH_3$ | $OCH_3$ | $CH_3$ | $C_2H_5$ | $NH(CH_2)_2$- 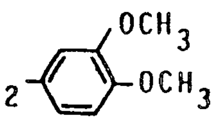 | 168-169 | HCl |
| 58 | H | $OCH_3$ | $OCH_3$ | $CH_3$ | $C_2H_5$ | $NH-(CH_2)_2$- 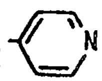 | 157-159 | HCl |
| 59 | H | $OCH_3$ | $OCH_3$ | $CH_3$ | $C_2H_5$ | NH- 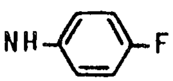 | 182-183 | HCl |
| 60 | H | $OCH_3$ | $OCH_3$ | $CH_3$ | $C_2H_5$ | $OC_2H_5$ | 164-166 | HCl |
| 61 | H | $OCH_3$ | $OCH_3$ | H | $(CH_2)_3-CH_3$ | $NHCH_3$ | 214-215 | HCl |
| 62 | H | $OCH_3$ | $OCH_3$ | H | $(CH_2)_3-CH_3$ | $NHC_2H_5$ | 203-204 | HCl |
| 63 | H | $OCH_3$ | $OCH_3$ | H | $(CH_2)_3-CH_3$ | $NH(CH_2)_2CH_3$ | 183-184 | BS |
| 64 | H | $OCH_3$ | $OCH_3$ | H | $(CH_2)_3-CH_3$ | $NH(CH_2)_3CH_3$ | 168-178 | HCl |
| 65 | H | $OCH_3$ | $OCH_3$ | H | $(CH_2)_3-CH_3$ | $NHCH_2CH(CH_3)_2$ | 186 | HCl |
| 66 | H | $OCH_3$ | $OCH_3$ | H | $(CH_2)_3-CH_3$ | $NHCH_2CH=CH_2$ | 191-192 | HCl |
| 67 | H | $OCH_3$ | $OCH_3$ | H | $(CH_2)_3CH_3$ | $NHCH(CH_3)_2$ | 197-199 | HCl |
| 68 | H | $OCH_3$ | $OCH_3$ | H | $(CH_2)_3CH_3$ | $NH(CH_2)_4CH_3$ | 183-186 | HCl |
| 69 | H | $OCH_3$ | $OCH_3$ | H | $(CH_2)_3CH_3$ | $NHCH(CH_3)CH_2CH_2CH_2CH(CH_3)_2$ | 190-193 | HCl |
| 70 | H | $OCH_3$ | $OCH_3$ | H | $(CH_2)_3CH_3$ | $NHCH_2CH(OH)CH_3$ | 130-135 | HCl |
| 71 | H | $OCH_3$ | $OCH_3$ | H | $(CH_2)_3CH_3$ | $NH(CH_2)_2OCH_3$ | 167-169 | HCl |
| 72 | H | $OCH_3$ | $OCH_3$ | H | $(CH_2)_3CH_3$ | $OC_2H_5$ | 141-145 | HCl |

| Nr. | $R^1$ | $R^2$ | $R^3$ | $R^4$ | $R^5$ | $R^6$ | Fp | Salzform |
|---|---|---|---|---|---|---|---|---|
| 73 | H | $OCH_3$ | $OCH_3$ | H | $(CH_2)_3CH_3$ | $NH(CH_2)_3OCH_3$ | 178-179 | HCl |
| 74 | H | $OCH_3$ | $OCH_3$ | H | $(CH_2)_3CH_3$ | $NH(CH_2)_2OH$ | 142-144 | HCl |
| 75 | H | $OCH_3$ | $OCH_3$ | H | $(CH_2)_3CH_3$ | $NH(CH_2)_2$- 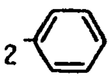 | 154-156 | HCl |
| 76 | H | $OCH_3$ | $OCH_3$ | H | $(CH_2)_3CH_3$ | $NH-(CH_2)_2$- 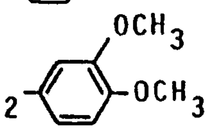 | 172-174 | HCl |
| 77 | H | $OCH_3$ | $OCH_3$ | H | $(CH_2)_3CH_3$ | $NH-(CH_2)_2$- 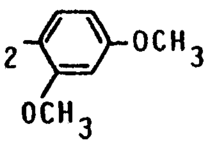 | 168-172 | HCl |
| 78 | H | $OCH_3$ | $OCH_3$ | H | $(CH_2)_3CH_3$ | $NH-CH_2$- 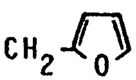 | 176-178 | HCl |
| 79 | H | $OCH_3$ | $OCH_3$ | H | $(CH_2)_3CH_3$ | $NH-(CH_2)_2$- 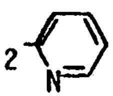 | 204-206 | HCl |
| 80 | H | $OCH_3$ | $OCH_3$ | H | $(CH_2)_3CH_3$ | $NH(CH_2)_2-N(CH_3)_2$ | 224-225 | HCl |
| 81 | H | $OCH_3$ | $OCH_3$ | H | $(CH_2)_3CH_3$ | $NH(CH_2)_3-N(CH_3)_2$ | 121-123 | BS |
| 82 | H | $OCH_3$ | $OCH_3$ | H | $(CH_2)_3CH_3$ | $N(C_2H_5)_2$ | 188-191 | HCl |
| 83 | H | $OCH_3$ | $OCH_3$ | H | $(CH_2)_3CH_3$ | $N(CH_2)_3CH_3)_2$ | 149-151 | HCl |
| 84 | H | $OCH_3$ | $OCH_3$ | H | $(CH_2)_3CH_3$ | 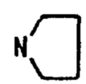 | 146-151 | HCl |
| 85 | H | $OCH_3$ | $OCH_3$ | H | $(CH_2)_3CH_3$ | 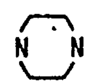 $-CH_2-CH_2$- 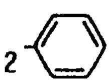 | 167-170 | HCl |

| Nr. | $R^1$ | $R^2$ | $R^3$ | $R^4$ | $R^5$ | $R^6$ | Fp | Salzform |
|---|---|---|---|---|---|---|---|---|
| 86 | H | $OCH_3$ | $OCH_3$ | H | $(CH_2)_3CH_3$ | 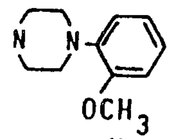 | 182-184 | HCl |
| 87 | H | $OCH_3$ | $OCH_3$ | H | $(CH_2)_3CH_3$ | 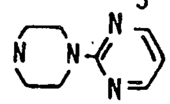 | 110-118 | HCl |
| 88 | H | $OCH_3$ | $OCH_3$ | H | $(CH_2)_3CH_3$ | $NHCH(CH_3)C_2H_5$ | 183-200 | HCl |
| 89 | H | $OCH_3$ | $OCH_3$ | H | $(CH_2)_3CH_3$ | $N(CH_3)_2$ | 192-194 | HCl |
| 90 | H | $OCH_3$ | $OCH_3$ | H | $(CH_2)_3CH_3$ | $NH(CH_2)_3OH$ | 168-170 | HCl |
| 91 | H | $OCH_3$ | $OCH_3$ | H | $(CH_2)_3CH_3$ | 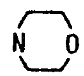 | 186-188 | HCl |
| 92 | H | $OCH_3$ | $OCH_3$ | H | $(CH_2)_3CH_3$ | $NH(CH_2)_2CH(CH_3)_2$ | 175-185 | HCl |
| 93 | H | $SCH_3$ | H | H | $(CH_2)_3CH_3$ | $OC_2H_5$ | 122-123 | HCl |
| 94 | H | $SCH_3$ | H | H | $(CH_2)_3CH_3$ | $NH(CH_2)_4CH_3$ | 94- 95 | BS |
| 95 | H | $OCH_3$ | $OCH_3$ | H | $(CH_2)_3CH_3$ | $N((CH_2)_2CH_3)_2$ | 179-180 | HCl |
| 96 | H | $OCH_3$ | $OCH_3$ | $CH_3$ | $(CH_2)_3CH_3$ | $NH(CH_2)_2$- 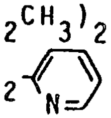 | 94- 96 | BS |
| 97 | H | $OCH_3$ | $OCH_3$ | $CH_3$ | $(CH_2)_3CH_3$ | $NH(CH_2)_2$- 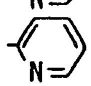 | 75- 78 | BS |
| 98 | H | $OCH_3$ | $OCH_3$ | $CH_3$ | $(CH_2)_3CH_3$ | $OC_2H_5$ | 156-157 | HCl |
| 99 | H | $OCH_3$ | $OCH_3$ | $CH_3$ | $(CH_2)_3CH_3$ | 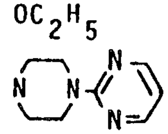 | 161-163 | BS |
| 100 | H | $OCH_3$ | $OCH_3$ | $CH_3$ | $(CH_2)_3CH_3$ | 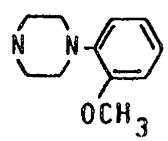 | 138-140 | HCl |

| Nr. | $R^1$ | $R^2$ | $R^3$ | $R^4$ | $R^5$ | $R^6$ | Fp | Salzform |
|---|---|---|---|---|---|---|---|---|
| 101 | H | $OCH_3$ | $OCH_3$ | $CH_3$ | $(CH_2)_3CH_3$ | 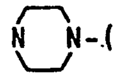 $-(CH_2)_2-$ 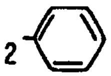 | 156-158 | HCl |
| 102 | H | $OCH_3$ | $OCH_3$ | $CH_3$ | $(CH_2)_3CH_3$ | $N((CH_2)_3CH_3)_2$ | 169-170 | HCl |
| 103 | H | $OCH_3$ | $OCH_3$ | $CH_3$ | $(CH_2)_3CH_3$ | OH | 180-183 | HCl |
| 104 | H | $OCH_3$ | $OCH_3$ | H | $(CH_2)_4CH_3$ | $OC_2H_5$ | 134-137 | HCl |
| 105 | H | $OCH_3$ | $OCH_3$ | H | $(CH_2)_4CH_3$ | $NH(CH_2)_4CH_3$ | 112-114 | BS |
| 106 | H | $OCH_3$ | $OCH_3$ | H | $(CH_2)_4CH_3$ | $NH(CH_2)_2CH(CH_3)_2$ | 172 | HCl |
| 107 | H | $OCH_3$ | $OCH_3$ | H | $(CH_2)_4CH_3$ | $NHC(CH_3)_3$ | 88- 90 | BS |
| 108 | H | $OCH_3$ | $OCH_3$ | H | $(CH_2)_4CH_3$ | $NH-CH_2CH=CH_2$ | 114-116 | BS |
| 109 | H | $OCH_3$ | $OCH_3$ | H | $(CH_2)_4CH_3$ | $NH-CH_2-$ 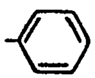 | 121-122 | BS |
| 110 | H | $OCH_3$ | $OCH_3$ | H | $(CH_2)_4CH_3$ | $NH-CH_2-$ 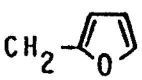 | 182-185 | HCl |
| 111 | H | $OCH_3$ | $OCH_3$ | H | $(CH_2)_4CH_3$ | NH- 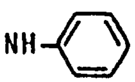 | 138-140 | BS |
| 112 | H | $OCH_3$ | $OCH_3$ | H | $(CH_2)_4CH_3$ | $N((CH_2)_2CH_3)_2$ | 152 | HCl |
| 113 | H | $OCH_3$ | $OCH_3$ | H | $(CH_2)_4CH_3$ | $NH(CH_2)_2-$ 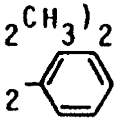 | 157 | BS |
| 114 | H | $OCH_3$ | $OCH_3$ | H | $(CH_2)_4CH_3$ | $NH(CH_2)_2N(CH_3)_2$ | 108 | BS |
| 115 | H | $OCH_3$ | $OCH_3$ | H | $(CH_2)_4CH_3$ | $NH-(CH_2)_2-$ 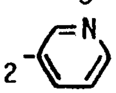 | 116-118 | BS |
| 116 | H | $OCH_3$ | $OCH_3$ | H | $(CH_2)_4CH_3$ | $NH-(CH_2)_2-$ 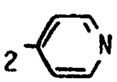 | 125 | BS |

| Nr. | $R^1$ | $R^2$ | $R^3$ | $R^4$ | $R^5$ | $R^6$ | Fp | Salzform |
|---|---|---|---|---|---|---|---|---|
| 117 | H | $OCH_3$ | $OCH_3$ | H | $(CH_2)_4CH_3$ | NH-$(CH_2)_2$- 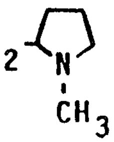 | 114-116 | BS |
| 118 | H | $OCH_3$ | OH | H | $C_6H_5$ | $NH(CH_2)_4CH_3$ | 153-160 | HCl |
| 119 | H | $OCH_3$ | OH | H | $C_6H_5$ | $NHCH_2CH=CH_2$ | 156-160 | HCl |
| 120 | H | $OCH_3$ | OH | H | $C_6H_5$ | $N(CH_2CH_2CN)CH_2-C_6H_5$ | 188-192 | HCl |
| 121 | H | $OCH_3$ | OH | H | $C_6H_5$ | NH-$(CH_2)_2$- 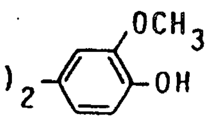 | 150-152 | BS |
| 122 | H | $OCH_3$ | OH | H | $C_6H_5$ | NH-$(CH_2)_2$- 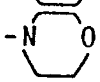 | 140-143 | BS |
| 123 | H | $OCH_3$ | H | H | $C_6H_5$ | $N(CH_3)C_2H_5$ | 138(Zers) | HCl |
| 124 | H | $OCH_3$ | H | H | $C_6H_5$ | NH-$CH_2$- 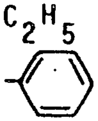 | 151 | BS |
| 125 | H | $OCH_3$ | H | H | $C_6H_5$ | $N(CH_3)_2$ | 136 | BS |
| 126 | H | $OCH_3$ | H | H | $C_6H_5$ | $NHC_2H_5$ | 153-154 | BS |
| 127 | H | $OCH_3$ | H | H | $C_6H_5$ | NH-$(CH_2)_2$- 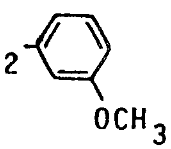 | 153 | BS |
| 128 | H | H | H | H | $C_6H_5$ | $NHCH_3$ | 159-160 | BS |
| 129 | $OCH_3$ | $OCH_3$ | $OCH_3$ | H | $C_6H_5$ | 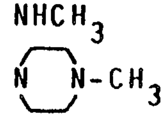 | ab 128 (Zers.) | HCl |
| 130 | $OCH_3$ | $OCH_3$ | $OCH_3$ | H | $C_6H_5$ | $NH-CH_2-C_6H_5$ | 162-163 | BS |

| Nr. | $R^1$ | $R^2$ | $R^3$ | $R^4$ | $R^5$ | $R^6$ | Fp | Salzform |
|---|---|---|---|---|---|---|---|---|
| 131 | $OCH_3$ | $OCH_3$ | $OCH_3$ | H | $C_6H_5$ | $NH(CH_2)_2$- 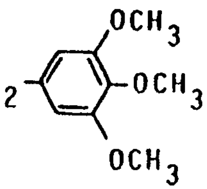 | 58- 59 | BS |
| 132 | H | $OCH_3$ | $OCH_3$ | H | $C_6H_5$ | $NH(CH_2)_2OH$ | 180-182 | HCl |
| 133 | H | $OCH_3$ | $OCH_3$ | H | $C_6H_5$ | 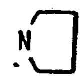 | 168-171 | HCl |
| 134 | H | $OCH_3$ | $OCH_3$ | H | $C_6H_5$ | $N(C_2H_5)_2$ | 125-127 | BS |
| 135 | H | $OCH_3$ | $OCH_3$ | H | $C_6H_5$ | $NH(CH_2)_2OCH_3$ | 172-175 | HCl |
| 136 | H | $OCH_3$ | $OCH_3$ | H | $C_6H_5$ | $NH(CH_2)_2$ 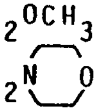 | 210-213 | HCl |
| 137 | H | $OCH_3$ | $OCH_3$ | H | $C_6H_5$ | 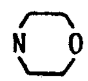 | 132-134 | BS |
| 138 | H | $OCH_3$ | $OCH_3$ | H | $C_6H_5$ | $NH-(CH_2)_2$- 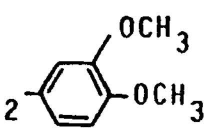 | 166-169 | HCl |
| 139 | H | $OCH_3$ | $OCH_3$ | $CH_3$ | $C_6H_5$ | $NH(CH_2)_2$- 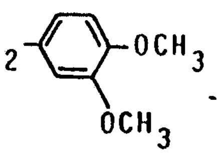 | 115-118 | BS |
| 140 | H | $OCH_3$ | $OCH_3$ | $CH_3$ | $C_6H_5$ | $N(C_2H_5)_2$ | 163 | BS |
| 141 | H | $OCH_3$ | $OCH_3$ | H | $C_6H_5(CH_2)_2$ | $OC_2H_5$ | 168 | HCl |
| 142 | H | $OCH_3$ | $OCH_3$ | H | $C_6H_5(CH_2)_2$ | $N[(CH_2)_3CH_3]_2$ | 152-154 | HCl |
| 143 | H | $OCH_3$ | $OCH_3$ | H | $C_6H_5(CH_2)_2$ | $NH(CH_2)_3CH_3$ | 117-119 | BS |
| 144 | H | $OCH_3$ | $OCH_3$ | H | $C_6H_5(CH_2)_2$ | $NH(CH_2)_2OH$ | 123-124 | BS |

| Nr. | $R^1$ | $R^2$ | $R^3$ | $R^4$ | $R^5$ | $R^6$ | Fp | Salzform |
|---|---|---|---|---|---|---|---|---|
| 145 | H | $OCH_3$ | $OCH_3$ | H | $C_6H_5(CH_2)_2$ | $NHCH(CH_3)(CH_2)_3CH(CH_3)_2$ | 144-146 | BS |
| 146 | H | $OCH_3$ | $OCH_3$ | H | $C_6H_5(CH_2)_2$ | $NH(CH_2)_2$- 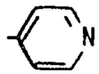 | 135-136 | BS |
| 147 | H | $OCH_3$ | $OCH_3$ | H | $C_6H_5(CH_2)_2$ | $NH(CH_2)_2$- 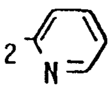 | 139-140 | BS |
| 148 | H | $OCH_3$ | $OCH_3$ | H | $C_6H_5(CH_2)_2$ | $NH(CH_2)_2$- 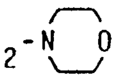 | 208-213 | HCl |
| 149 | H | $OCH_3$ | $OCH_3$ | H | $C_6H_5(CH_2)_2$ | $NH(CH_2)_2C_6H_5$ | 119-121 | BS |
| 150 | H | $OCH_3$ | $OCH_3$ | H | $C_6H_5(CH_2)_2$ | $NHCH_2C_6H_5$ | 129-132 | BS |
| 151 | H | $OCH_3$ | $OCH_3$ | H | $C_6H_5(CH_2)_2$ | $NHC_6H_5$ | 162-164 | BS |
| 152 | H | $OCH_3$ | $OCH_3$ | H | $C_6H_5(CH_2)_2$ | $NH-CH_2$- 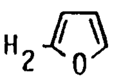 | 141-144 | BS |
| 153 | H | $OCH_3$ | $OCH_3$ | H | $C_6H_5(CH_2)_2$ | NH- 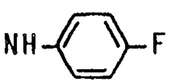 | 156-159 | BS |
| 154 | H | $OCH_3$ | $OCH_3$ | H | $C_6H_5(CH_2)_2$ | $NH(CH_2)_2$- 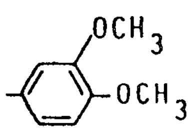 | 129 | BS |
| 155 | H | $OCH_3$ | $OCH_3$ | H | $C_6H_5(CH_2)_2$ | $NH(CH_2)_2$- 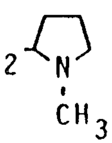 | 124-126 | BS |

| Nr. | $R^1$ | $R^2$ | $R^3$ | $R^4$ | $R^5$ | $R^6$ | Fp | Salzform |
|---|---|---|---|---|---|---|---|---|
| 156 | H | $OCH_3$ | $OCH_3$ | $CH_3$ | $C_6H_5(CH_2)_2$ | $NH(CH_2)_2$- 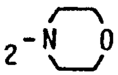 | 105-106 | BS |
| 157 | H | $OCH_3$ | $OCH_3$ | $CH_3$ | $C_6H_5(CH_2)_2$ | $NH(CH_2)_2$- 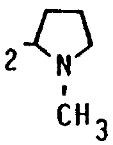 | 106-108 | BS |
| 158 | H | $OCH_3$ | $OCH_3$ | H | $OCH_3$ | $OC_2H_5$ | 214-217 | HCl |
| 159 | H | $OCH_3$ | $OCH_3$ | H | $OCH_3$ | $NH(CH_2)_2CH_3$ | 162 | HCl |
| 160 | H | $OCH_3$ | $OCH_3$ | H | $OCH_3$ | $NH(CH_2)_4CH_3$ | 122-123 | HCl |
| 161 | H | $OCH_3$ | $OCH_3$ | H | $OCH_3$ | $NHCH_2CH(CH_3)_2$ | 174 | HCl |
| 162 | H | $OCH_3$ | $OCH_3$ | H | $OCH_3$ | $NH-CH_2CH=CH_2$ | 137 | HCl |
| 163 | H | $OCH_3$ | $OCH_3$ | H | $OCH_3$ | $NH-CH-C\equiv CH$ | 130 | HCl |
| 164 | H | $OCH_3$ | $OCH_3$ | H | $OCH_3$ | $NH-(CH_2)_2-OCH_3$ | 87 | HCl |
| 165 | H | $OCH_3$ | $OCH_3$ | H | $OCH_3$ | $NH(CH_2)_2$- 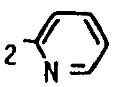 | 89- 90 | HCl |
| 166 | H | $OCH_3$ | $OCH_3$ | H | $OCH_3$ | $NH(CH_2)_2$- 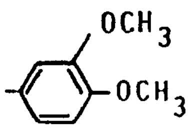 | 71- 75 | BS |
| 167 | H | $OCH_3$ | $OCH_3$ | H | $OCH_3$ | $OC_2H_5$ | 182-184 | HCl |
| 168 | H | $OCH_3$ | $OCH_3$ | $CH_3$ | $(CH_2)_3CH_3$ | $NH(CH_2)_2$- 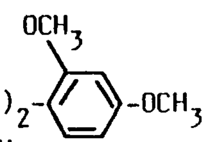 | 94-96 | BS |
| 169 | H | $OCH_3$ | $OCH_3$ | $CH_3$ | $(CH_2)_3CH_3$ | $NH(CH_2)_2$- 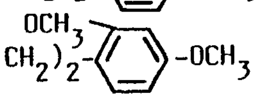 | 75-78 | BS |

Tabelle 2:

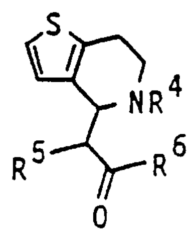

| Nr. | $R_4$ | $R_5$ | $R_6$ | Fp | Salzform |
|---|---|---|---|---|---|
| 170 | H | $C_6H_5$ | $NH(CH_2)_2$ 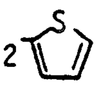 | 171-172 | HCl |
| 171 | $CH_3$ | $C_6H_5$ | $NH(CH_2)$- 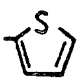 | 101-105 | BS |
| 172 | H | $C_6H_5$ | $NH(CH_2)_2$- 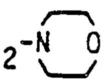 | 128 (Zers.) | BS |
| 173 | H | $C_6H_5$ | 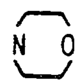 | 210-215 | HCl |
| 174 | H | $C_6H_5(CH_2)_2$ | $N(C_2H_5)_2$ | 191-193 | HCl |
| 175 | H | $C_6H_5(CH_2)_2$ | 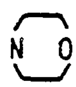 | 145 (Zers.) | HCl |

Tabelle 3:

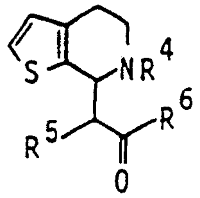

| | | | | | |
|---|---|---|---|---|---|
| 176 | H | $C_6H_5$ | 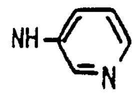 | 175 (Zers.) | HCl |
| 177 | H | $C_6H_5$ | 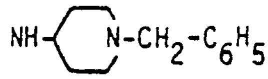 | 195 (Zers.) | HCl |
| 178 | H | $C_6H_5$ | 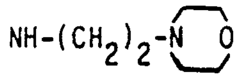 | 90 (Zers.) | HCl |

Tabelle 4:

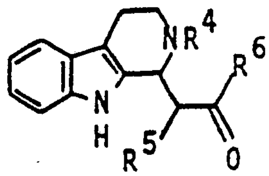

| Nr. | $R_4$ | $R_5$ | $R_6$ | Fp | Salzform |
|---|---|---|---|---|---|
| 179 | H | $C_6H_5$ | 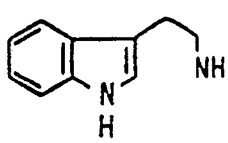 | 197-200 | HCl |
| 180 | H | H | 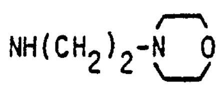 | 262-265 | HCl |
| 181 | H | H | $NH-CH_2CH(CH_3)_2$ | 165-168 | BS |
| 182 | H | H | $OC_2H_5$ | 128-130 | BS |

Tabelle 5

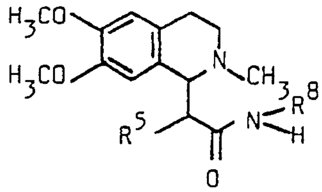

| Nr. | $R^8$ | $R^5$ | Fp (°C) | Salzform |
|---|---|---|---|---|
| 183 | $CH_3-CH_2-CH_2-$ | H | 95-96 | BS |
| 184 | $CH_3-$ | H | 96-100 | BS |
| 185 | $CH_2=CH-CH_2-$ | H | 87-91 | BS |
| 186 | $CH_3O-CH_2-CH_2-$ | H | ÖL | |
| 187 | $CH_3-(CH_2)_4-$ | H | 87-90 | BS |
| 188 | $CH_3-(CH_2)_3-$ | H | 74-75 | BS |
| 189 | $(CH_3)_2N-(CH_2)_3-$ | H | 170-190 (Zersetzung) | HCl |
| 190 | $(CH_3)_2CH-CH_2-$ | H | 84-90 | BS |
| 191 | $(CH_3)_2CH-$ | H | 144-145 | BS |
| 192 | $CH_3O-(CH_2)_3-$ | H | 71-75 | BS |
| 193 | $CH_3-CH_2-CH_2-$ | $CH_3$ | 119-124 | BS |
| 194 | 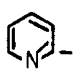 $-CH_2-CH_2-$ | $CH_3$ | 96-102 | BS |
| 195 | $CH_3-(CH_2)_4-$ | $C_2H_5$ | 93-95 | BS |
| 196 | 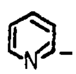 $-CH_2-CH_2-$ | $C_2H_5$ | 86-89 | BS |
| 197 | $(CH_3)_2CH-CH_2-$ | $C_2H_5$ | 110-112 | BS |
| 198 | 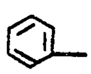 | $C_5H_{11}$ | 123-125 | BS |

Tabelle 6

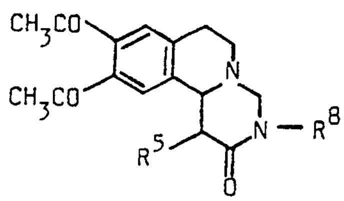

| Nr. | $R^8$ | $R^5$ | Fp. (°C) | Salzform |
|---|---|---|---|---|
| 199 | $-CH_2-CH_2-CH_3$ | H | | HCl |
| 200 | $-CH_3$ | H | 226-228 | HCl |
| 201 | $-CH_2-CH=CH_2$ | H | 183-186 | HCl |
| 202 | $-CH_2-CH_2-OCH_3$ | H | 198-203 | HCl |
| 203 | $-CH_2-CH_2-N(CH_3)_2$ | H | 173-176 | HCl |
| 204 | $-CH_2-CH_2-CH_2-CH_2-CH_3$ | H | 177-179 | HCl |
| 205 | $-CH_2-CH_2-CH_2-CH_3$ | H | 189-191 | HCl |
| 206 | $-CH_2-CH_2-CH_2-N(CH_3)_2$ | H | 168-172 | HCl |
| 207 | $-CH_2-CH_3$ | H | 218-222 | HCl |
| 208 | $-CH_2-CH(CH_3)_2$ | H | 189-194 | HCl |
| 209 | $-CH(CH_3)_2$ | H | 209-211 | HCl |
| 210 | $-CH_2-CH_2-CH(CH_3)_2$ | H | 182-185 | HCl |
| 211 | $-CH_2-CH_2-CH_2-OCH_3$ | H | 206-208 | HCl |
| 212 | $-CH_2CH_2CH_3$ | $CH_3$ | | HCl |
| 213 | $-CH_2CH_2-$ 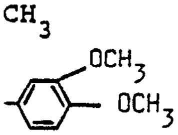 | $CH_3$ | 181-184 | HCl |
| 214 | $-CH_2-CH(CH_3)_2$ | $CH_3$ | 194-195 | HCl |
| 215 | 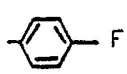 | $CH_3$ | 170-172 | HCl |
| 216 | $-CH_2CH_2OCH_3$ | $CH_3$ | 173-175 | HCl |
| 217 | $-CH_2CH_2CH_2CH_2CH_3$ | $CH_3$ | 168-170 | HCl |
| 218 | $-CH_2CH_2-$ 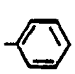 | $CH_3$ | 190-194 | HCl |
| 219 | $-CH_2CH_2-$ 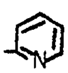 | $CH_3$ | 197-200 | HCl |

| Nr. | $R^8$ | $R^5$ | Fp. (°C) | Salzform |
|---|---|---|---|---|
| 220 | $-CH_2CH_2-$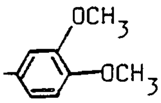 | $C_2H_5$ | 159-164 | HCl |
| 221 | $-CH_2CH_2CH_2CH_2CH_3$ | $C_2H_5$ | 124-133 | HCl |
| 222 | $CH_2CH_2-$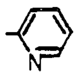 | $C_2H_5$ | 192-202 | HCl |
| 223 | 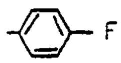 | $C_2H_5$ | 157-162 | HCl |
| 224 | $CH_2CH_2OCH_3$ | $C_2H_5$ | 171-175 | HCL |
| 225 | $-CH(CH_3)CH_2CH_2CH_2CH(CH_3)_2$ | $C_2H_5$ | 151-159 | HCl |
| 226 | $-CH_2CH_2-$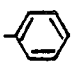 | $C_2H_5$ | 178-184 | HCl |
| 227 | $-CH_2CH_2-$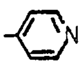 | $C_2H_5$ | 192-193 | HCl |
| 228 | $-CH_2CH(CH_3)_2$ | $C_2H_5$ | 164-167 | HCl |
| 229 | $-CH_2CH=CH_2$ | $n-C_4H_9$ | 76-78(A) | HCl |
| 230 | $-CH_2-CH_2-N(CH_3)_2$ | $n-C_4H_9$ | 181 | HCl |
| 231 | $-CH_2CH_2OCH_3$ | $n-C_4H_9$ | 47-62(A) | HCl |
| 232 | $-CH_2CH_2-$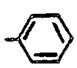 | $n-C_4H_9$ | 111-118 | HCl |
| 233 | $-CH_2CH(CH_3)_2$ | $n-C_4H_9$ | 95-96(A) | HCl |
| 234 | $-CH_2CH_2CH_2CH_2CH_3$ | $n-C_4H_9$ | 68-73(A) | HCl |
| 235 | $-CH_2CH_2CH_3$ | $n-C_4H_9$ | 54-57(A) | HCl |
| 236 | $-CH_2CH_2CH(CH_3)_2$ | $n-C_4H_9$ | 90-92(A) | HCl |
| 237 | $-CH_2CH_2-$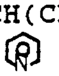 | $n-C_4H_9$ | 197-198 | HCl |
| 238 | 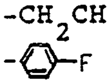 | $n-C_4H_9$ | 150-152 | HCl |
| 239 | 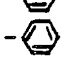 | $n-C_5H_{11}$ | 134-138 | BS |
| 240 | $-CH_2-$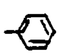 | $n-C_5H_{11}$ | 107-109 | BS |

| Nr. | $R^8$ | $R^5$ | Fp. (°C) | Salzform |
|---|---|---|---|---|
| 241 | $-CH_2-CH_2-$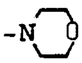 | 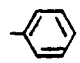 | 151 | |
| 242 | $-CH_2-$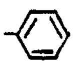 | $-OCH_3$ | 155 | HCl |
| 243 | $-CH_2CH_2-$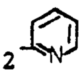 | $-OCH_3$ | 127-128 | BS |
| 244 | $-CH_2CH_2-N(CH_3)_2$ | $n-C_4H_9$ | | HCl |

(A): amorphes Isomerengemisch

| Verbindung Nr. | Isomerengemisch |
|---|---|
| 229 | 50:50 |
| 231 | 70:30 |
| 233 | 40:60 |
| 234 | 90:10 |
| 235 | 60:40 |
| 236 | 70:30 |

## Pharmazeutische Anwendungsbeispiele

| a) Dragées | |
|---|---|
| 1 Drageekern enthält: | |
| Wirkstoff der allgemeinen Formel I oder V | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

### Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40 °C getrocknet und nochmals durch ein Sieb getrieben. Das so erhaltene Granulat wird mir Magnesiumstearat gemischt und verpreßt. Die so erhaltenen Kerne werden in üblicher Weise mit einer Hülle überzogen, die mit Hilfe einer wässerigen

Suspension von Zucker, Titandioxyd, Talkum und Gummi arabicum aufgebracht wird. Die fertigen Dragées werden mit Bienenwachs poliert.

| b) Tabletten | |
|---|---|
| Wirkstoff der allgemeinen Formel I oder V | 30,0 mg |
| Milchzucker | 100,0 mg |
| Maisstärke | 70,0 mg |
| lösliche Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff der allgemeinen Formel I oder V | 20,0 mg |
| Milchzucker | 230,0 mg |
| Maisstärke | 40,0 mg |
| Talk | 10,0 mg |
| | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

## Ansprüche

1. Verwendung einer Verbindung der allgemeinen Formel I,

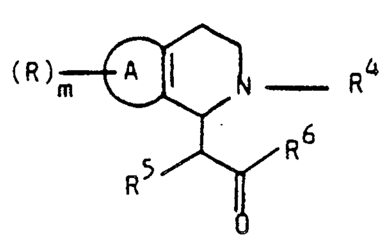

I

worin
A einen Benzo-, Thieno- oder Indolorest bedeutet;
R Wasserstoff, $(C_1-C_4)$Alkyl, Halogen (F, Cl, Br, J), Hydroxy, $(C_1-C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;

m 1, 2 oder 3 bedeutet, wenn A ein Benzo- oder Indolorest ist, und 1 oder 2 bedeutet, wenn A ein Thienorest ist;
$R^4$ Wasserstoff oder $(C_1\text{-}C_4)$Alkyl bedeutet;
$R^5$ Wasserstoff, $(C_1\text{-}C_{10})$Alkyl, Phenyl, Phenyl$(C_1\text{-}C_5)$alkyl, $(C_1\text{-}C_4)$Alkoxy oder -NHCOX (worin X $(C_1\text{-}C_5)$-Alkyl ist) bedeutet;
$R^6$ Hydroxy, $(C_1\text{-}C_4)$Alkoxy oder eine $-NR^7R^8$-Gruppe bedeutet,
worin $R^7$ und $R^8$ unabhängig voneinander
(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch
Hydroxy,
$(C_1\text{-}C_4)$Alkoxy,
Di$(C_1\text{-}C_4)$Alkylamino,
Furyl,
Pyridyl,
Pyrrolidinyl,
Morpholino,
Indolyl,
Nitrilo,
Thienyl,
Phenyl oder Phenyl, das ein oder mehrfach durch Hydroxy, Methoxy oder Fluor substituiert ist;
oder $R^7$ Wasserstoff bedeutet und $R^8$ Phenyl, Fluorophenyl, Pyridyl oder N-Benzylpiperidyl;
oder $R^7$ und $R^8$ gemeinsam mit dem Stickstoffatom, an das sie gebunden sind,
Pyrrolidinyl
Piperidinyl
Morpholinyl- oder
Piperazinyl bedeuten, wobei der Piperazinylring gegebenenfalls durch Methyl, unsubstituiertes Phenyl, Mono-oder Di$(C_1\text{-}C_4)$alkoxyphenyl,
Pyrimidinyl oder Phenyl$(C_1\text{-}C_4)$alkyl N-substituiert sein kann;
oder deren pharmazeutisch annehmbaren Salzes mit einer anorganischen oder organischen Säure zur Herstellung eines Mittels zur Kardioprotektion.

2. Pharmazeutische Zubereitung enthaltend
i) eine Verbindung der Formel I, die wie in Anspruch 1 definiert ist, oder deren pharmazeutisch annehmbare Salze mit anorganischen oder organischen Säuren,
ausgenommen
a) Verbindungen der Formel Ig,

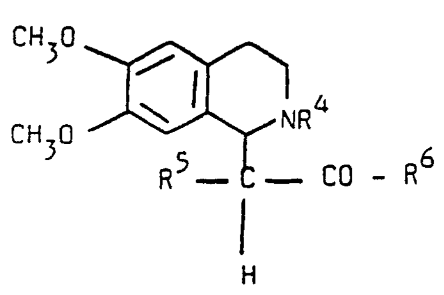

(Ig)

worin
$R^4$ H ist,
$R^5$ H ist und
$R^6$ $NH(CH_2)_3OH$,
$NH(CH_2)_2OH$ oder
$NH(CH_2)_3OCH_3$ ist;
b) Verbindungen der Formel I, worin
A ein Indolorest ist;
R Wasserstoff, $(C_1\text{-}C_4)$Alkyl, Halogen oder $(C_1\text{-}C_4)$Alkoxy bedeutet;

m 1 ist,
$R^4$ Wasserstoff oder $(C_1-C_4)$Alkyl bedeutet,
$R^5$ Wasserstoff bedeutet,
$R^6$ $(C_1-C_4)$Alkoxy oder eine $NHR^8$-Gruppe bedeutet, worin
$R^8$ Wasserstoff oder Alkyl mit 1 bis 4 Kohlenstoffatomen (das durch Phenyl substituiert sein kann) bedeutet;
oder ii) eine Verbindung der allgemeinen Formel Ih,

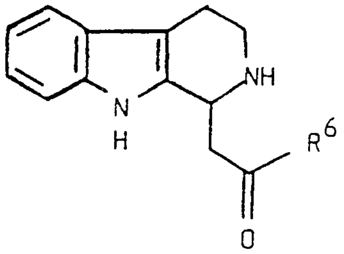

(Ih)

worin $R^6$ Ethoxy oder $-NHCH_2CH(CH_3)_2$ ist.

3. Eine Verbindung der Formel I

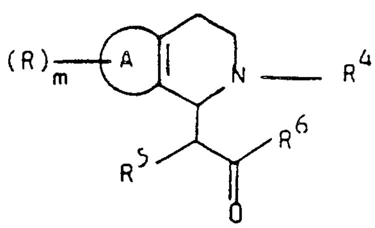

(I)

oder deren pharmazeutisch annehmbares Salz mit einer anorganischen oder organischen Säure, wobei A, R, m, $R^4$, $R^5$ und $R^6$ wie in Anspruch 1 definiert sind, ausgenommen

a) Verbindungen der Formel Ic,

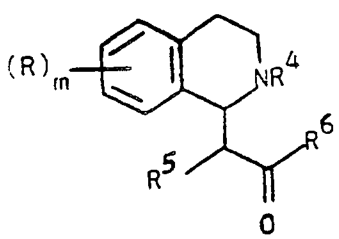

(Ic)

worin
R Wasserstoff, Hydroxy oder Methoxy ist,
m 1, 2 oder 3 ist,
$R^4$ und $R^5$ Wasserstoff sind und
$R^6$ Hydroxy oder Ethoxy ist;
sowie Verbindungen, worin
m 2 ist und
R Methoxy in den Positionen 6 und 7, und
$R^4$ Wasserstoff, $R^5$ Ethyl und $R^6$ Methoxy ist, oder
$R^4$ Methyl, $R^5$ Wasserstoff und $R^6$ Hydroxy oder Ethoxy ist,
oder
$R^4$ Wasserstoff, $R^5$ Methyl und $R^6$ Ethoxy ist;
sowie Verbindungen der Formel Id,

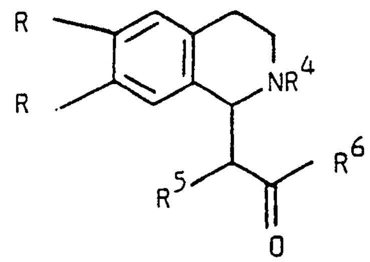

(Id)

worin R Wasserstoff oder Methoxy ist,
$R^4$ Methyl,
$R^5$ Wasserstoff oder Phenyl und
$R^6$ Ethoxy ist;

b) Verbindungen der Formel Ie,

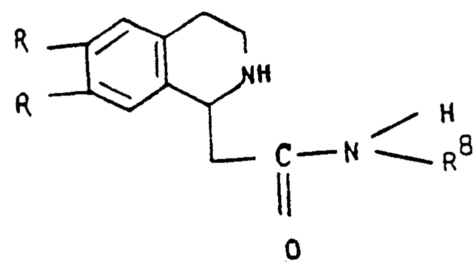

(Ie)

worin
R Wasserstoff, $(C_1-C_4)$Alkyl, Hydroxy oder $(C_1-C_4)$Alkoxy bedeutet, oder die zwei benachbarten Substituenten R zusammen $-O-CH_2-O-$sind,
$R^8$ Wasserstoff, Alkenyl, Alkyl, das durch Hydroxy, Methoxy, Dimethylamin oder Phenyl substituiert sein kann, bedeutet;

c) Verbindungen der Formel If,

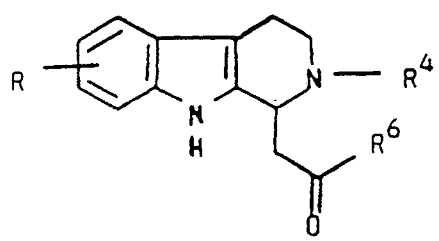

(If)

worin
R Wasserstoff, $(C_1-C_4)$Alkyl, Halogen oder $(C_1-C_4)$Alkoxy, bedeutet,
$R^4$ Wasserstoff oder $(C_1-C_4)$Alkyl bedeutet,
$R^6$ $(C_1-C_4)$Alkoxy oder eine $-NHR^8$-Gruppe bedeutet,
worin
$R^8$ Wasserstoff oder $(C_1-C_4)$-Alkyl bedeutet,
wobei das Alkyl substituiert sein kann durch Phenyl.

4. Verwendung einer Verbindung nach Anspruch 1, in der A ein Benzorest ist und vorzugsweise:
-) R Wasserstoff, Hydroxy, $(C_1-C_4)$Alkoxy oder Methylthio ist, vorzugsweise Methoxy;
-) insbesondere R Methoxy und m 2 ist und die Substituenten R in den Positionen 6 und 7 sind;
-) $R^4$ Wasserstoff oder Methyl ist;
-) $R^5$ Wasserstoff, $(C_1-C_5)$Alkyl, Phenyl, Phenyl$(C_1-C_5)$alkyl oder $(C_1-C_4)$Alkoxy ist; vorzugsweise $R^5$ Wasserstoff, $(C_1-C_5)$Alkyl, Phenyl, Phenethyl oder Methoxy ist, vorzugsweise $C_4$- oder $C_5$-Alkyl oder Phenethyl;
-) $R^6$ Hydroxy oder Ethoxy ist; oder

-) $R^6$ eine $NR^7R^8$-Gruppe ist; in der vorzugsweise
-) $R^7$ und $R^8$ $(C_1-C_5)$Alkyl sind; oder
-) $R^7$ Wasserstoff ist und $R^8$ Alkenyl oder Alkinyl mit 3 Kohlenstoffatomen, vorzugsweise $-CH_2CH=CH_2$; oder
-) $R^7$ Wasserstoff ist und $R^8$ Alkyl mit 1-8 Kohlenstoffatomen; oder
-) $R^7$ Wasserstoff und $R^8$ Alkyl mit 1 bis 3 Kohlenstoffatomen, das substituiert sein kann durch Hydroxy, Methoxy, Dimethylamino, Furyl, Pyridyl, Pyrrolidinyl, Morpholino, Phenyl oder Penyl das 1 bis 3 fach substituiert ist durch Hydroxy oder Methoxy; oder
-) $R^7$ und $R^8$ gemeinsam mit dem Stickstoffatom, an das sie gebunden sind, Pyrrolidinyl, Morpholino oder Piperazinyl bedeuten.

5. Verwendung einer Verbindung nach Anspruch 1, in der A eine Thienorest ist und R Wasserstoff und vorzugsweise
-) $R^4$ Wasserstoff oder Methyl ist;
-) $R^5$ Phenyl oder Phenethyl ist;
-) $R^6$ eine $-NR^7R^8$-Gruppe ist, worin vorzugsweise
-) $R^7$ Wasserstoff, Methyl oder Ethyl ist und $R^8$ Methyl oder Ethyl, des durch Morpholino oder Thienyl substituiert sein kann; oder
-) $R^7$ und $R^8$ gemeinsam mit dem Stickstoffatom, an das sie gebunden sind, Morpholino sind.

6. Pharmazeutische Zubereitung nach Anspruch 2 enthaltend eine Verbindung der Formel I, die wie in Anspruch 4 oder 5 definiert ist.

7. Verbindung der Formel I nach Anspruch 3, worin A, R, m, $R^4$, $R^5$ und $R^6$ wie in Anspruch 4 oder 5 definiert sind.

8. Verfahren zur Herstellung einer Verbindung der allgemeinen Formel I, dadurch gekennzeichnet, daß man
zur Herstellung einer Verbindung der allgemeinen Formel I nach Anspruch 3 oder 7 eine Verbindung II,

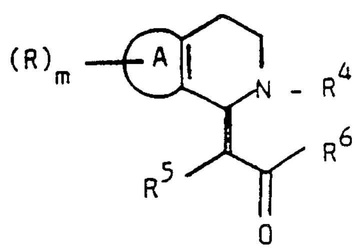

(II)

in der A, R, m, $R^4$, $R^5$ und $R^6$ wie in der gewünschten Verbindung der Formel I definiert sind, reduziert oder
zur Herstellung einer Verbindung der allgemeinen Formel I, worin $R^4$ die Gruppe $-CR^9H_2$ ist,
$R^6$ die Gruppe $-NHR^8$ ist und
A einen Benzo-, Thieno- oder Indolorest bedeutet;
R Wasserstoff, $(C_1-C_4)$Alkyl, Halogen (F, Cl, Br, J), Hydroxy, $(C_1-C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;
m 1, 2 oder 3 bedeutet, wenn A ein Benzo- oder Indolorest ist, und 1 oder 2 bedeutet, wenn A ein Thienorest ist;
$R^9$ Wasserstoff oder $(C_1-C_3)$Alkyl bedeutet;
$R^5$ Wasserstoff, $(C_1-C_{10})$Alkyl, Phenyl, Phenyl$(C_1-C_5)$alkyl, $(C_1-C_4)$Alkoxy oder -NHCOX (worin X $(C_1-C_5)$-Alkyl ist) bedeutet;
$R^8$ (a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeutet, wobei das Alkyl substituiert sein kann durch
Hydroxy, $(C_1-C_4)$Alkoxy, Di$(C_1-C_4)$Alkylamino, Furyl, Pyridyl, Pyrrolidinyl, Morpholino, Indolyl, Nitrilo, Thienyl, Phenyl oder Phenyl, das ein oder mehrfach durch Hydroxy, Methoxy oder Fluor substituiert ist;
(e) Phenyl, Fluorophenyl, Pyridyl oder N-Benzylpiperidyl bedeutet;
eine Verbindung der allgemeinen Formel V,

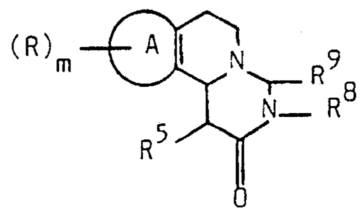

V

in der A, m, R, $R^5$, $R^8$ und $R^9$ wie in der gewünschten Verbindung der Formel I definiert ist, reduziert; und die so erhaltene Verbindung gewünschtenfalls einer oder mehreren der folgenden Nachbehandlungen unterwirft:

a) Überführung einer Verbindung der allgemeinen Formel I, in der $R^6$ Hydroxy oder $(C_1$-$C_4)$Alkoxy ist, in das entsprechende Amid, in dem $R^6$ die Gruppe $NR^7R^8$ ist;

b) N-Alkylierung einer Verbindung der allgemeinen Formel I, in der $R^4$ Wasserstoff ist, zu der entsprechenden Verbindung der Formel I, in der $R^4$ $(C_1$-$C_4)$Alkyl ist;

c) Überführung einer Verbindung der Formel I, in der $R^6$ Hydroxy ist, in den entsprechenden Ester ($R^6$ = $(C_1$-$C_4)$Alkoxy);

d) Hydrolyse einer Verbindung der Formel I, in der $R^6$ $(C_1$-$C_4)$Alkoxy oder $NR^7R^8$ ist, zu der entsprechenden freien Säure;

e) Isolierung der einzelnen Diastereomeren;

f) Isolierung der Verbindung in freier Form oder in Form ihres Säureadditionssalzes.

9. Eine Verbindung der Formel II

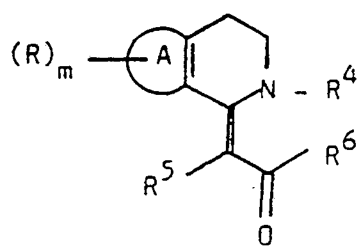

II

oder deren Salz mit einer anorganischen oder organischen Säure, worin
A ein Indolrest ist,
R, m, $R^4$, $R^5$ und $R^6$ wie in Anspruch 1 definiert sind, ausgenommen Verbindungen, worin
m eins ist,
R Wasserstoff, $(C_1$-$C_4)$Alkyl, Halogen oder $(C_1$-$C_4)$Alkoxy bedeutet,
$R^4$ Wasserstoff oder $(C_1$-$C_4)$Alkyl bedeutet,
$R^5$ Wasserstoff bedeutet,
$R^6$ $(C_1$-$C_4)$Alkoxy oder eine -$NHR^8$-Gruppe bedeutet,
worin
$R^8$ Wasserstoff oder $(C_1$-$C_4)$-Alkyl bedeutet, wobei das Alkyl substituiert sein kann durch Phenyl.

10. Verfahren zur Herstellung einer Verbindung der Formel II nach Anspruch 9, dadurch gekennzeichnet, daß man ein Malonsäurediamid der allgemeinen Formel IV,

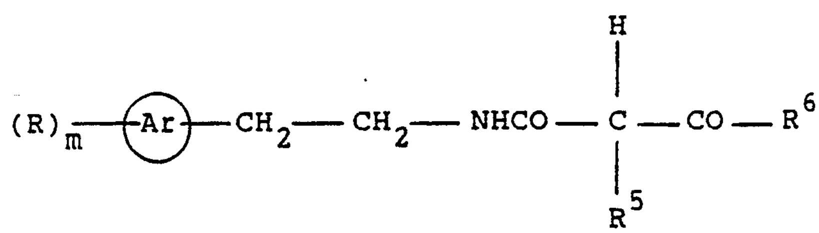

(IV)

in der R, m, $R^5$ und $R^6$ wie in der gewünschten Verbindung der Formel II definiert sind und Ar 2-oder 3-Indolyl bedeutet zu der entsprechenden Verbindung II cyclisiert.

11. Verbindung der allgemeinen Formel V

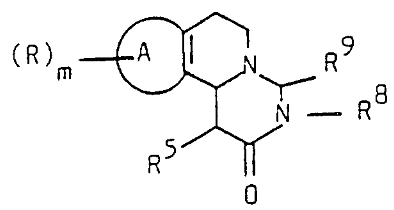

V

worin

A einen Benzo-, Thieno- oder Indolorest bedeutet;

R Wasserstoff, $(C_1-C_4)$Alkyl, Halogen (F, Cl, Br, J), Hydroxy, $(C_1-C_4)$Alkoxy, Amino, Methylthio, Methansulfonyloxy oder Methansulfonamido bedeutet, oder zwei benachbarte Substituenten R zusammen $-O-CH_2-O-$ oder $-O-CH_2-CH_2-O-$ sind;

m 1, 2 oder 3 bedeutet, wenn A ein Benzo- oder Indolorest ist, und 1 oder 2 bedeutet, wenn A ein Thienorest ist;

$R^9$ Wasserstoff oder $(C_1-C_3)$Alkyl bedeutet;

$R^5$ Wasserstoff, $(C_1-C_{10})$Alkyl, Phenyl, Phenyl$(C_1-C_5)$alkyl, $(C_1-C_4)$Alkoxy oder -NHCOX (worin X $(C_1-C_5)$-Alkyl ist) bedeutet;

$R^8$ (a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeutet,

wobei das Alkyl substituiert sein kann durch

Hydroxy,

$(C_1-C_4)$Alkoxy,

Di$(C_1-C_4)$Alkylamino,

Furyl,

Pyridyl,

Pyrrolidinyl,

Morpholino,

Indolyl,

Nitrilo,

Thienyl,

Phenyl oder Phênyl, das ein oder mehrfach durch Hydroxy, Methoxy oder Fluor substituiert ist;

(e) Phenyl, Fluorophenyl, Pyridyl oder N-Benzylpiperidyl; bedeutet

und deren Salze mit einer anorganischen oder organischen Säure,

vorzugsweise eine Verbindung der Formel V, worin A, R, m, $R^5$ und $R^8$ wie in Anspruch 4 oder 5 definiert sind.

12. Verfahren zur Herstellung einer Verbindung nach Anspruch 11, dadurch gekennzeichnet, daß man eine entsprechende Verbindung der allgemeinen Formel I, worin $R^4$ Wasserstoff ist und $R^6$ die Gruppe $-NHR^8$ bedeutet und R, m, $R^5$ und $R^8$ wie in der gewünschten Verbindung der Formel V definiert sind (diese Verbindung entspricht der allgemeinen Formel I'), mit einem Aldehyd der allgemeinen Formel $R^9CHO$ (VI) umsetzt

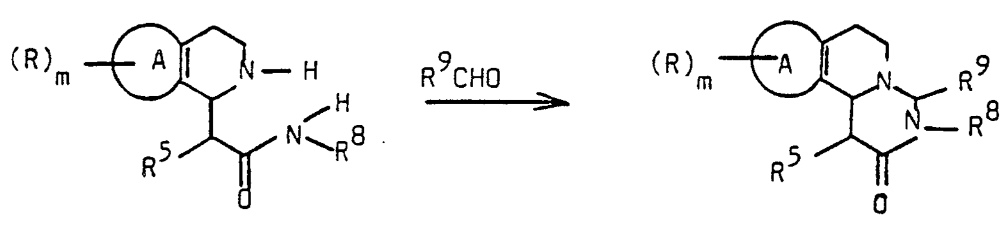


I' V

worin $R^9$ Wasserstoff oder $(C_1-C_3)$Alkyl bedeutet.

13. Pharmazeutische Zubereitung enthaltend eine Verbindung der Formel V, die wie in Anspruch 11

definiert ist, oder deren pharmazeutisch annehmbares Salz mit einer anorganischen oder organischen Säure.

Europäisches Patentamt

# EUROPÄISCHER RECHERCHENBERICHT

Nummer der Anmeldung

EP 89 11 4943

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 251 194 (BOEHRINGER INGELHEIM KG)<br>* Ansprüche 1-10 *<br>--- | 1,2,4,5,7,9,10 | C 07 D 217/16<br>C 07 D 471/04<br>C 07 D 495/04<br>C 07 D 471/14<br>C 07 D 495/14<br>A 61 K 31/47<br>A 61 K 31/435//<br>(C 07 D 471/04<br>C 07 D 221:00<br>C 07 D 209:00 )<br>(C 07 D 471/04<br>C 07 D 239:00<br>C 07 D 221:00 )<br>(C 07 D 495/04<br>C 07 D 333:00<br>C 07 D 221:00 ) |
| X,P | EP-A-0 288 048 (BOEHRINGER INGELHEIM KG)<br>* Ansprüche 1,12-14; Seiten 20-32 *<br>--- | 1,4-7,10 | |
| X | EP-A-0 015 786 (SYNTHELABO)<br>* Seite 1; Beispiele 2,3 *<br>--- | 11,12 | |
| A,D | US-A-3 021 331 (J. G. LOMBARDINO et al.)<br>* Ansprüche 1,10 *<br>--- | 3,11,12 | |
| A,D | EP-A-0 021 857 (SYNTHELABO)<br>* Zusammenfassung; Seiten 12-14; Tabelle *<br>--- | 3,9 | |
| A | DD-A- 139 126 (G. BERNATH et al.)<br>* Seite 3, Formeln (I),(VIII) *<br>--- | 3 | **RECHERCHIERTE SACHGEBIETE (Int. Cl.5)** |
| A,D | EUROPEAN JOURNAL OF MEDICINAL CHEMISTRY. CHIMICA THEREPEURICA<br>Band 14, Nr. 1, 1979, Seiten 77-84;<br>J.B. STENLAKE et al.: "Biodegradable neuromuscular blocking agents. I. Quaternary esters" * Seite 78, Verbindung 10; Seite 79, Verbindung 12 *<br>--- | 3 | A 61 K 31/00<br>C 07 D 217/00<br>C 07 D 471/00<br>C 07 D 495/00 |
| A,D | HETEROCYCLES<br>Band 3, Nr. 2, 1975, Seiten 1979-1982;<br>T. KAMETANI et al.: "A total synthesis of (+-)-yohimbine" * Seite 181, Verbindung (1) *<br>----- | 3 | |

Der vorliegende Recherchenbericht wurde für alle Patentansprüche erstellt

| Recherchenort | Abschlußdatum der Recherche | Prüfer |
|---|---|---|
| BERLIN | 10-11-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)